# EUROPEAN PATENT SPECIFICATION

(11) **EP 2 539 432 B1**
(45) Date of publication and mention of the grant of the patent: **12.04.2017**
(21) Application number: 11748040.0
(22) Date of filing: 24.02.2011
(51) Int. Cl.: C12N 1/20, A01N 63/02, A01N 43/74, A01N 43/14, A01N 43/16, A01N 43/76, A01N 63/00, C07D 309/14, C07D 407/06, C07D 413/04, C07D 413/06, C07D 263/34, C07D 263/32, C07D 498/14, C07D 513/04, C12P 17/16, A01N 43/90, C07D 493/10

(54) **ISOLATED BACTERIAL STRAIN OF THE GENUS BURKHOLDERIA AND PESTICIDAL METABOLITES THEREFROM**
ISOLIERTER BAKTERIENSTRANG DES STAMMES BURKHOLDERIA UND PESTIZIDE METABOLITE DARAUS
SOUCHE BACTÉRIENNE ISOLÉE DU GENRE BURKHOLDERIA ET MÉTABOLITES PESTICIDES ISSUS DE CETTE SOUCHE

(30) Priority: 25.02.2010 US 308287 P; 25.10.2010 US 406541 P
(43) Date of publication of application: 02.01.2013
(73) Proprietor: Marrone Bio Innovations, Inc., Davis, CA 95618 (US)
(72) Inventor: ASOLKAR, Ratnakar, Davis, CA 95618 (US); KOIVUNEN, Marja, Davis, CA 95618 (US); MARRONE, Pamela, Davis, CA 95616 (US); HUANG, Huazhang, Woodland, CA 95776 (US); CORDOVA-KREYLOS, Ana Lucia, Davis, CA 95616 (US)
(74) Representative: Kjerrumgaard, Lars Bo
(86) International application number: PCT/US2011/026016
(87) International publication number: WO 2011/106491

(56) References cited:
- WO-A1-01/55143
- WO-A1-97/20857
- WO-A1-2009/049378
- WO-A2-2005/115149
- JP-A- 2007 091 701
- KR-B1- 100 537 389
- US-A1- 2003 082 147
- US-A1- 2004 071 663
- US-A1- 2008 096 879
- DATABASE EMBL [Online] 7 June 2000 (2000-06-07), XP002696963, Database accession no. AF148554
- DATABASE EMBL [Online] 8 June 2001 (2001-06-08), XP002696964, Database accession no. AF265235
- DATABASE EMBL [Online] 2 April 2003 (2003-04-02), XP002696965, Database accession no. AB092606
- DATABASE EMBL [Online] 27 November 2001 (2001-11-27), XP002696966, Database accession no. AJ420880
- DATABASE EMBL [Online] 5 September 2000 (2000-09-05), XP002696967, Database accession no. AF175314
- DATABASE EMBL [Online] 20 November 2007 (2007-11-20), XP002696968, Database accession no. AM905038
- DATABASE EMBL [Online] 10 October 2004 (2004-10-10), XP002696969, Database accession no. AY740337
- DATABASE EMBL [Online] 28 March 2006 (2006-03-28), XP002696970, Database accession no. AB212236
- DATABASE EMBL [Online] 28 March 2006 (2006-03-28), XP002696971, Database accession no. AB212227
- DATABASE EMBL [Online] 10 October 2004 (2004-10-10), XP002696972, Database accession no. AY741345
- DATABASE EMBL [Online] 2 July 2009 (2009-07-02), XP002696973, Database accession no. AB508854
- DATABASE EMBL [Online] 10 October 2004 (2004-10-10), XP002696974, Database accession no. AY741351
- DATABASE EMBL [Online] 10 October 2004 (2004-10-10), XP002696975, Database accession no. AY741349
- DATABASE EMBL [Online] 31 August 2005 (2005-08-31), XP002696976, Database accession no. AY40350
- DATABASE EMBL [Online] 10 October 2004 (2004-10-10), XP002696977, Database accession no. AY741334
- DATABASE EMBL [Online] 10 October 2004 (2004-10-10), XP002696978, Database accession no. AY741348
- DATABASE EMBL [Online] 10 October 2004 (2004-10-10), XP002696979, Database accession no. AY741330
- DATABASE EMBL [Online] 10 October 2004 (2004-10-10), XP002696980, Database accession no. AY741340
- DATABASE EMBL [Online] 10 October 2004 (2004-10-10), XP002696981, Database accession no. AY741339
- DATABASE EMBL [Online] 27 June 2007 (2007-06-27), XP002696982, Database accession no. AM747631
- DATABASE EMBL [Online] 10 October 2004 (2004-10-10), XP002696983, Database accession no. AY741359
- DATABASE EMBL [Online] 10 October 2004 (2004-10-10), XP002696984, Database accession no. AY741341
- DATABASE EMBL [Online] 10 October 2004 (2004-10-10), XP002696985, Database accession no. AY741353
- DATABASE EMBL [Online] 21 June 2007 (2007-06-21), XP002696986, Database accession no. AM747632
- DATABASE EMBL [Online] 21 June 2007 (2007-06-21), XP002696987, Database accession no. AM747628
- DATABASE EMBL [Online] 3 August 2004 (2004-08-03), XP002696988, Database accession no. AY661910
- DATABASE EMBL [Online] 3 June 2009 (2009-06-03), XP002696989, Database accession no. FJ932759
- DATABASE EMBL [Online] 21 June 2007 (2007-06-21), XP002696990, Database accession no. AM747630
- DATABASE EMBL [Online] 8 June 2008 (2008-06-08), XP002696991, Database accession no. EU684748
- DATABASE EMBL [Online] 22 January 1999 (1999-01-22), XP002696992, Database accession no. AB021369
- DATABASE EMBL [Online] 10 October 2004 (2004-10-10), XP002696993, Database accession no. AY741361
- DATABASE EMBL [Online] 10 October 2004 (2004-10-10), XP002696994, Database accession no. AY741335
- DATABASE EMBL [Online] 16 April 2005 (2005-04-16), XP002696995, Database accession no. AB211225
- DATABASE EMBL [Online] 29 December 2008 (2008-12-29), XP002696996, Database accession no. FJ436055
- DATABASE EMBL [Online] 7 December 2005 (2005-12-07), XP002696997, Database accession no. DQ273265
- DATABASE EMBL [Online] 16 August 2009 (2009-08-16), XP002696998, Database accession no. GQ359110
- DATABASE EMBL [Online] 10 July 1998 (1998-07-10), XP002696999, Database accession no. U96927
- DATABASE EMBL [Online] 3 November 2008 (2008-11-03), XP002697000, Database accession no. EU826644
- DATABASE EMBL [Online] 8 October 1997 (1997-10-08), XP002697001, Database accession no. E10021
- DATABASE EMBL [Online] 29 August 2006 (2006-08-29), XP002697002, Database accession no. AB252073
- DATABASE EMBL [Online] 1 July 1998 (1998-07-01), XP002697003, Database accession no. U96928
- DATABASE EMBL [Online] 8 January 2008 (2008-01-08), XP002697004, Database accession no. EU305400
- DATABASE EMBL [Online] 10 May 2009 (2009-05-10), XP002697005, Database accession no. FJ870663
- DATABASE EMBL [Online] 3 August 2004 (2004-08-03), XP002697006, Database accession no. AY662003
- DATABASE EMBL [Online] 17 June 2003 (2003-06-17), XP002697007, Database accession no. AJ491304
- DATABASE EMBL [Online] 1 July 1998 (1998-07-01), XP002697008, Database accession no. U96937
- DATABASE EMBL [Online] 1 July 1998 (1998-07-01), XP002697009, Database accession no. U96929
- DATABASE EMBL [Online] 20 January 2009 (2009-01-20), XP002697010, Database accession no. FJ606689
- DATABASE EMBL [Online] 26 March 2005 (2005-03-26), XP002697011, Database accession no. AY946011
- DATABASE EMBL [Online] 8 July 2008 (2008-07-08), XP002697012, Database accession no. EU214612
- DATABASE EMBL [Online] 26 March 2005 (2005-03-26), XP002697013, Database accession no. AY946010

### SEQUENCE LISTING

<110> Marrone Bio Innovations, Inc.
<120> ISOLATED BACTERIAL STRAIN OF THE GENUS BURKHOLDERIA
<130> MOI-42016-US
<150> 61308287
   <151> 2010-02-25
<150> 61406541
   <151> 2010-10-25
<160> 15
<170> PatentIn version 3.5
<210> 1
   <211> 20
   <212> DNA
   <213> primer
<400> 1
   agagtttgat cctggctcag 20
<210> 2
   <211> 20
   <212> DNA
   <213> primer
<400> 2
   ccgtcaattc ctttgagttt 20
<210> 3
   <211> 16
   <212> DNA
   <213> primer
<400> 3
   gtgccagccg ccgcgg 16
<210> 4
   <211> 16
   <212> DNA
   <213> primer
<400> 4
   gcaacgagcg caaccc 16
<210> 5
   <211> 17
   <212> DNA
   <213> primer
<400> 5
   aaggaggtgw tccarcc 17
<210> 6
   <211> 15
   <212> DNA
   <213> primer
<400> 6
   gggttgcgct cgttg 15
<210> 7
   <211> 18
   <212> DNA
   <213> primer
<400> 7
   gwattaccgc ggckgctg 18
<210> 8
   <211> 871
   <212> DNA
   <213> Burkholderia A396
<400> 8
   tgcagtcgaa cggcagcacg ggtgcttgca cctggtggcg agtggcgaac gggtgagtaa 60
   tacatcggaa catgtcctgt agtgggggat agcccggcga aagccggatt aataccgcat 120
   acgatctacg gatgaaagcg ggggatcttc ggacctcgcg ctatagggtt ggccgatggc 180
   tgattagcta gttggtgggg taaaggccta ccaaggcgac gatcagtagc tggtctgaga 240
   ggacgatcag ccacactggg actgagacac ggcccagact cctacgggag gcagcagtgg 300
   ggaattttgg acaatggggg aaaccctgat ccagcaatgc cgcgtgtgtg aagaaggcct 360
   tcgggttgta aagcactttt gtccggaaag aaatcctttg ggctaatacc ccggggggat 420
   gacggtaccg gaagaataag caccggctaa ctacgtgcca gcagccgcgg taatacgtag 480
   ggtgcgagcg ttaatcggaa ttactgggcg taaagcgtgc gcaggcggtt tgttaagaca 540
   gatgtgaaat ccccgggctt aacctgggaa ctgcatttgt gactggcaag ctagagtatg 600
   gcagaggggg gtagaattcc acgtgtagca gtgaaatgcg tagagatgtg gaggaatacc 660
   gatggcgaag gcagccccct gggccaatac tgacgctcat gcacgaaagc gtggggagca 720
   aacaggatta gataccctgg tagtccacgc cctaaacgat gtcaactagt tgttggggat 780
   tcatttcctt agtaacgtag ctacgcgtga agttgaccgc ctggggagta cggtcgcaag 840
   attaaatmga gggtkgkktg kkggggggaa a 871
<210> 9
   <211> 1453
   <212> DNA
   <213> Burkholderia A396
<400> 9
   gtcatgaatc ctaccgtggt gaccgtcctc cttgcggtta gactagccac ttctggtaaa 60
   acccactccc atggtgtgac gggcggtgtg tacaagaccc gggaacgtat tcaccgcggc 120
   atgctgatcc gcgattacta gcgattccag cttcatgcac tcgagttgca gagtgcaatc 180
   cggactacga tcggttttct gggattagct ccccctcgcg ggttggcaac cctctgttcc     240
   gaccattgta tgacgtgtga agccctaccc ataagggcca tgaggacttg acgtcatccc     300
   caccttcctc cggtttgtca ccggcagtct ccttagagtg ctcttgcgta gcaactaagg     360
   acaagggttg cgctcgttgc gggacttaac ccaacatctc acgacacgag ctgacgacag     420
   ccatgcagca cctgtgtatc ggttctcttt cgagcactcc cgaatctctt caggattccg     480
   accatgtcaa gggtaggtaa ggtttttcgc gttgcatcga attaatccac atcatccacc     540
   gcttgtgcgg gtccccgtca attcctttga gttttaatct tgcgaccgta ctccccaggc     600
   ggtcaacttc acgcgttagc tacgttacta aggaaatgaa tccccaacaa ctagttgaca     660
   tcgtttaggg cgtggactac cagggtatct aatcctgttt gctccccacg ctttcgtgca     720
   tgagcgtcag tattggccca gggggctgcc ttcgccatcg gtattcctcc acatctctac     780
   gcatttcact gctacacgtg gaattctacc cccctctgcc atactctagc ttgccagtca     840
   caaatgcagt tcccaggtta agcccgggga tttcacatct gtcttaacaa accgcctgcg     900
   cacgctttac gcccagtaat tccgattaac gctcgcaccc tacgtattac cgcggctgct     960
   ggcacgtagt tagccggtgc ttattcttcc ggtaccgtca tccccccggg gtattagccc    1020
   aaaggatttc tttccggaca aaagtgcttt acaacccgaa ggccttcttc acacacgcgg    1080
   cattgctgga tcagggtttc ccccattgtc caaaattccc cactgctgcc tcccgtagga    1140
   gtctgggccg tgtctcagtc ccagtgtggc tgatcgtcct ctcagaccag ctactgatcg    1200
   tcgccttggt aggcctttac cccaccaact agctaatcag ccatcggcca accctatagc    1260
   gcgaggtccg aagatccccc gctttcatcc gtagatcgta tgcggtatta atccggcttt    1320
   cgccgggcta tcccccacta caggacatgt tccgatgtat tactcacccg ttcgccactc    1380
   gccaccaggt gcaagcaccc gtgctgccgt tcgacttgca tgtgtaaggc atgccgccag    1440
   cgttcaatct gag                                                        1453
<210> 10
   <211> 860
   <212> DNA
   <213> Burkholderia A396
<400> 10
   ccaggcggtc acttcacgcg ttagctacgt tactaaggaa atgaatcccc aacaactagt        60
   tgacatcgtt tagggcgtgg actaccaggg tatctaatcc tgtttgctcc ccacgctttc       120
   gtgcatgagc gtcagtattg gcccaggggg ctgccttcgc catcggtatt cctccacatc       180
   tctacgcatt tcactgctac acgtggaatt ctacccccct ctgccatact ctagcttgcc        240
   agtcacaaat gcagttccca ggttaagccc ggggatttca catctgtctt aacaaaccgc       300
   ctgcgcacgc tttacgccca gtaattccga ttaacgctcg caccctacgt attaccgcgg       360
   ctgctggcac gtagttagcc ggtgcttatt cttccggtac cgtcatcccc ccggggtatt       420
   agcccaaagg atttctttcc ggacaaaagt gctttacaac ccgaaggcct tcttcacaca       480
   cgcggcattg ctggatcagg gtttccccca ttgtccaaaa ttccccactg ctgcctcccg       540
   taggagtctg ggccgtgtct cagtcccagt gtggctgatc gtcctctcag accagctact       600
   gatcgtcgcc ttggtaggcc tttaccccac caactagcta atcagccatc ggccaaccct       660
   atagcgcgag gtccgaagat cccccgcttt catccgtaga tcgtatgcgg tattaatccg       720
   gctttcgccg ggctatcccc cactacagga catgttccga tgtattactc acccgttcgc       780
   cactcgccac caggtgcaag cacccgtgct gccgttcgac ttgcatgtgt aaggcatgcc       840
   gccagcgttc aatctgagtg                                                   860
<210> 11
   <211> 1152
   <212> DNA
   <213> Burkholderia A396
<400> 11
   tcggattact gggcgtaagc gtgcgcaggc ggtttgttaa gacagatgtg aaatccccgg      60
   gcttaacctg ggaactgcat ttgtgactgg caagctagag tatggcagag gggggtagaa     120
   ttccacgtgt agcagtgaaa tgcgtagaga tgtggaggaa taccgatggc gaagggagcc     180
   ccctgggcct atactgaccc tcatgctcga aagcgtgagg acccaaccgg attagatgcc     240
   ctgataggcc atgccccaca ccatgccatg tgttaggggc ccatttcctt agggaggcag     300
   ctatggggaa ttttggacaa tgtgggaaac cctgatccaa caatgccgcg tgtgtgaata     360
   aggccttcgg gttgtaaagc acttttatcc ggatagattc cttttgggct aaacctccgt     420
   aggggatgac ggtaccggaa gaataaccac cgggtaacta cgtgccagca gccgcggtaa     480
   tacgtagggt gcgagcgtta atcggaatta ctgggcgtaa agcgtgcgca ggcggtttgt     540
   taagacagat gtgaaatccc cgggcttaac ctgggaactg catttgtgac tggcaagcta     600
   gagtatggca gacgggggta gaattccacg tgtagcagtg aaatgcgtag agatgtggag     660
   gaataccgat gggcgaagca gctcctgggg caatactgac gctcatgcac aagatcgtgc     720
   gaaacaaaca ggataaaacc cctgtattcc acgcccaaaa cgatgtccac caagttgttg     780
   gcgatccttt ccttcgtatc gtagctacgc gggaatttga ccccctgggg actaggccgc     840
   atataaaact caagggaatt ccggggaccc ccagagctgt gtatgatgtg attattccga     900
   tgcgcggaaa accttcctta tctttgaatg gcggtactcc tgaaaattgc ggagtgctcg     960
   aaaacaccga acccgggtct ttctgcgtgt cctccctcgt gtgggatatg ctggatatcc    1020
   cgcagacgca tctttgactt agtgctccca aaactgagag ctgggaggac tcgagagggg    1080
   atccctgcct ccccggcttg ggtgctcccc ttatggggga aacaggtaca cggggggatc    1140
   atcccatacc ta                                                        1152
<210> 12
   <211> 1067
   <212> DNA
   <213> Burkholderia A396
<400> 12
   tctaaggaga ctgccggtga caaaccggag gaaggtgggg atgacgtcaa gtcctcatgg        60
   cccttatggg tagggcttca cacgtcatac aatggtcgga acagagggtt gccaacccgc       120
   gagggggagc taatcccaga aaaccgatcg tagtccggat tgcactctgc aactcgagtg       180
   catgaagctg gaatcgctag taatcgcgga tcagcatgcc gcggtgaata cgttcccggg       240
   tcttgtacac accgcccgtc acaccatggg agtgggtttt accagaagtg gctagtctaa       300
   ccgcaaggag gacggtcacc acggtaggat tcatgactgg ggtgaagtcg taacaaggta       360
   gccgtatcgg aaggtgcggc tggatcacct ccttaaaccc tttggcctaa taaccccggg       420
   ggaataagta ccgaaaaaaa aaaaaactgg ataacttccg tgccacaacc cgcggaaaaa       480
   tctagggggg gggagcttaa atggaaattt acggggccgt aaagcgtgcg caggcggttt       540
   gtaaacacag atgtgaaatc cccgggctta acctgggaac tgcatttgtg actggcaagc       600
   tagagtatgg cacagggggg tagaattcca cgtgtagcat tgaatgcata gagatgagag       660
   gataccgatg gagaagggcg cccccgggga caatatgacg cctatgccac aaagctgtgg       720
   cacaataggt taaatacctg tgttgtcccc gcctaaacag attacacttg ttgtgggtat       780
   tttctcataa aatactacac acgggagaat acactggggg gcttcgtcaa ttatcacaac       840
   aatgattgcg ggcacccacg ggggtagatg ggtaataaat cgacggcaac tatctactta       900
   cttggatgat cgcacagatt gggcgggaga gaagagaaca gcgtgtgtgt gctcctccgc       960
   gagtgatagg taatcggaca atactttgac aggacttaac tgggtagcgg gatcgagtgg      1020
   attcccgtcg gatggcctcc gcaggtacgg cagctgggga ttacatc                    1067
<210> 13
   <211> 1223
   <212> DNA
   <213> Burkholderia A396
<400> 13
   ttgcttacga cttcacccca gtcatgaatc ctaccgtggt gaccgtcctc cttgcggtta        60
   gactagccac ttctggtaaa acccactccc atggtgtgac gggcggtgtg tacaagaccc       120
   gggaacgtat tcaccgcggc atgctgatcc gcgattacta gcgattccag cttcatgcac       180
   tcgagttgca gagtgcaatc cggactacga tcggttttct gggattagct ccccctcgcg       240
   ggttggcaac cctctgttcc gaccattgta tgacgtgtga agccctaccc ataagggcca       300
   tgaggacttg acgtcatccc caccttcctc cggtttgtca ccggcagtct ccttagagtg       360
   ctcttgcgta gcaactaagg acaagggttg cgctcgttgc gggacttaac ccaacatctc       420
   acgacacgag ctgacgacag ccatgcagca cctgtgtatc ggttctcttt cgagcactcc       480
   cgaatctctt caggattccg accatgtcaa gggtaggtaa ggtttttcgc gttgcatcga       540
   attaatccac atcatccacc gcttgtgcgg gtccccgtca attcctttga gttttaatct       600
   tgcgaccgta ctccccaggc ggtcaacttc acgcgttagc tacgttacta aggaaatgaa       660
   tccccaacaa ctagttgaca tcgtttaggg cgtggactac cagggtatct aatcctgttt       720
   gctccccacg ctttcgtgca tgagcgtcag tattggccca gggggctgcc ttcgccatcg       780
   gtattcctcc acatctctac gcatttcact gctacacgtg gaattctacc cccctctgcc       840
   atactctagc ttgccagtca caaatgcagt tcccaggtta agcccgggga tttcacatct       900
   gtcttaacaa accgcctgcg cacgctttac gcccagtaat tccgattaac gctcgcaccc       960
   tacgtattac cgcggctgct ggcacgtagt tagccggtgc ttattctgcg gtaccgtcat      1020
   cccccgggta tagcccaaag gattctttcg acaaagtgct ttacacccga tgtctctcac      1080
   acacgcgcat gctgatcagg tttccccatg tcaaagtcca ctgctgctcg taggtctgga      1140
   cgggttcagt tcaatgtgac tgatcgtctt tcgacaacta ctgaacgtcc ctgtagctta      1200
   cccaccaact agctatagca tgc                                              1223
<210> 14
   <211> 1216
   <212> DNA
   <213> Burkholderia A396
<400> 14
   ccgagctgac gacagccatg cagcacctgt gtatcggttc tctttcgagc actcccgaat 60
   ctcttcagga ttccgaccat gtcaagggta ggtaaggttt ttcgcgttgc atcgaattaa 120
   tccacatcat ccaccgcttg tgcgggtccc cgtcaattcc tttgagtttt aatcttgcga 180
   ccgtactccc caggcggtca acttcacgcg ttagctacgt tactaaggaa atgaatcccc 240
   aacaactagt tgacatcgtt tagggcgtgg actaccaggg tatctaatcc tgtttgctcc 300
   ccacgctttc gtgcatgagc gtcagtattg gcccaggggg ctgccttcgc catcggtatt 360
   cctccacatc tctacgcatt tcactgctac acgtggaatt ctacccccct ctgccatact 420
   ctagcttgcc agtcacaaat gcagttccca ggttaagccc ggggatttca catctgtctt 480
   aacaaaccgc ctgcgcacgc tttacgccca gtaattccga ttaacgctcg caccctacgt 540
   attaccgcgg ctgctggcac gtagttagcc ggtgcttatt cttccggtac cgtcatcccc 600
   ccggggtatt agcccaaagg atttctttcc ggacaaaagt gctttacaac ccgaaggcct 660
   tcttcacaca cgcggcattg ctggatcagg gtttccccca ttgtccaaaa ttccccactg 720
   ctgcctcccg taggagtctg ggccgtgtct cagtcccagt gtggctgatc gtcctctcag 780
   accagctact gatcgtcgcc ttggtaggcc tttaccccac caactagcta atcagccatc 840
   ggccaaccct atagcgcgag gtccgaagat cccccgcttt catccgtaga tcgtatgcgg 900
   tattaatccg gctttcgccg ggctatcccc cactacagga catgttccga tgtattactc 960
   acccgttcgc cactcgcccc aggtgcaagc acccgtgctg ccgttcgact tgcatgtgta 1020
   gcatgcgcag cgtcatctac taaataaaca actctaagaa tttttgcccg agggcctcta 1080
   aacactcggg gcgtcgagag agactacgga tgaggagcat ccctctgtct ctaggtatgt 1140
   gttgtcgcct ctctcacaga ggaggggacg cacgacggag ccatcgggga cgacaacatg 1200
   tacgatatac tatcta 1216
<210> 15
   <211> 1194
   <212> DNA
   <213> Burkholderia A396
<400> 15
   ttcttcggta ccgtcatccc cccggggtat tagcccaaag gatttctttc cggacaaaag      60
   tgctttacaa cccgaaggcc ttcttcacac acgcggcatt gctggatcag ggtttccccc     120
   attgtccaaa attccccact gctgcctccc gtaggagtct gggccgtgtc tcagtcccag     180
   tgtggctgat cgtcctctca gaccagctac tgatcgtcgc cttggtaggc ctttacccca     240
   ccaactagct aatcagccat cggccaaccc tatagcgcga ggtccgaaga tcccccgctt     300
   tcatccgtag atcgtatgcg gtattaatcc ggctttcgcc gggctatccc ccactacagg     360
   acatgttccg atgtattact cacccgttcg ccactcgcca ccaggtgcaa gcacccgtgc     420
   tgccgttcga cttgcatgtg taaggcatgc cgccagcgtt caatctgagc catgatcaaa     480
   ctctgagggg gggggccttc aacggaacga ctgggcaaaa agcgtgccca ggcgttttgt     540
   taagacagat gtgaaacccc ggggcttaac ctggaaactg catttgtgac tggaaagcta     600
   gagtatggca gaggggggta gaattccacg tgtagcattg aaatgcgtag aaatggagag     660
   gaataccgat gggagagggc agcccccgtg ggcaaatact ggcgcttatg aacaaagttg     720
   gggcgcgccg ccgggatatg ttcccctggg atatcccccc cctaaactgc ttacaaatat     780
   tgtgtgggaa actttttctc taaaaaatag aacacaacgg gagatatcac ccccgggggg     840
   ccaccgccag attaaacccc caaaaagtat ttggcgggca cccccccggg gggtgagatg     900
   gggtaaaata aatccgtgcg acgagcaaac cctccccaca cctgggatgg tcgcgaccac     960
   agatgagatg cgggcggaga gaacgatacc caagcgtggt tgtttgcctg catcccctcc    1020
   gtcgggagtg gatatagtag agtaattacg gcacgactgc attttttttt cttcagtaca    1080
   ccttatcaca ctgttggatg caccgcgaga aatccggagg tgtgagtact ccccccctct   1140
   cctcgggatg tgtcggcgct cccttctccc gttcaggggt gggtaagcac cgcg          1194

## Description

### TECHNICAL FIELD

Provided herein is a species of *Burkholderia sp* with no known pathogenicity to vertebrates, such as mammals, fish and birds but pesticidal activity against plants, insects, fungi and nematodes. Also provided are natural products derived from a culture of said species and methods of controlling germination and growth of dicotyledenous, monocotyledonous and sedge weeds, modulating growth of fungi and controlling pests such as insects and nematodes using said natural products.

### BACKGROUND

Natural products are substances produced by microbes, plants, and other organisms. Microbial natural products offer an abundant source of chemical diversity, and there is a long history of utilizing natural products for pharmaceutical purposes. One such compound is FR901228 isolated from *Chromobacterium* and has been found to be useful as an antibacterial agent and antitumor agent (see, for example, Ueda et al., US Patent No. 7,396,665).

However, secondary metabolites produced by microbes have also been successfully found to have uses for weed and pest control in agricultural applications (see, for example, Nakajima et al.1991; Duke et al., 2000; Lydon & Duke, 1999; Gerwick et al., US Patent No. 7,393,812). Microbial natural products have been also successfully developed into agricultural insecticides (see, for example, Salama et al. 1981; Thompson et al., 2000; Krieg et al. 1983). Sometimes, such natural products have been combined with chemical pesticides (see, for example, Gottlieb, US Patent No. 4,808,207).

WO01/55143 concerns azole and azine derivatives, processes for preparing them, fungicidal, insecticidal, acaricidal, molluscicidal and nematicidal compositions comprising them, methods of using them to combat fungal diseases (especially fungal diseases of plants) and methods of using them to combat and control insect, acarine, mollusc and nematode pests. The compounds herein are different from the compounds of the present invention in that templazole A has no linker between the indole and oxazole moieties and the oxazole is linked to the pyrrole moiety of the indole.

WO 2009049318 concerns bioactive molecules. More particularly, the invention relates to lactone derivatives useful as pharmaceutical, agricultural or pesticidal agents. The compounds herein are different from the compounds of the present invention in that no further amide-propenyl-carboxy-ethyl moiety is linked to the tetrahydropyrane ring.

WO 9720857 concerns a novel organic chemical compound, which is referred to below as Omphalotin. The compound is useful as a microbicide and pesticide, preferably for controlling animal pests, fungi and bacteria. The compounds herein are different from the compounds of the present invention in that it concerns a peptidic 12-membered ring substituted by an indole whereas FR90128 of the present invention is a hexapeptidic ring with a further S-S-butenyl linked inside of the ring.

JP2007091701 concerns compounds containing the structure amide-phenyl-dihydrooxazol and a further ring for use as pesticides. These are different from the present invention in that the templazole B has a further isobutyl substituent and an oxazole moiety.

WO2005115149 concerns a cyclic peptide isolated from an extract of bark of a Madagascan plant, which peptide has insecticidal activity.

### Burkholderia

The Burkholderia genus, β-subdivision of the proteobacteria, comprises more than 40 species that inhabit diverse ecological niches (Compant et al., 2008). The bacterial species in the genus *Burkholderia* are ubiquitous organisms in soil and rhizosphere (Coenye and Vandamme, 2003; Parke and Gurian-Sherman, 2001). Traditionally, they have been known as plant pathogens, *B. cepacia* being the first one discovered and identified as the pathogen causing disease in onions (Burkholder, 1950). Several *Burkholderia* species have developed beneficial interactions with their plant hosts (see, for example, Cabballero-Mellado et al., 2004, Chen et al., 2007). Some *"Burkholderia* species have also been found to be opportunistic human pathogens (see, for example, Cheng and Currie, 2005 and Nierman et al., 2004). Additionally, some *Burkholderia* species have been found to have potential as biocontrol products (see for example, Burkhead et al.,1994; Knudsen et al., 1987; Jansiewicz et al.,1988; Gouge et al., US Patent Application No. 2003/0082147; Parke et al., US Patent No. 6,077,505; Casida et al., US Patent No. 6,689,357; Jeddeloh et al., WO2001055398; Zhang et al., US Patent No. 7,141,407). Some species of in this genus have been effective in bioremediation to decontaminate polluted soil or groundwater (see, for example, Leahy et al. 1996). Further, some *"Burkholderia* species have been found to secrete a variety of extracellular enzymes with proteolytic, lipolytic and hemolytic activities, as well as toxins, antibiotics, and siderophores (see, for example, Ludovic et al., 2007; Nagamatsu, 2001).

### Oxazoles, Thiazoles and Indoles

Oxazoles, thiazoles and indoles are widely distributed in plants, algae, sponges, and microorganisms. A large number of natural products contain one or more of the five-membered oxazole, thiazole and indole nucleus/moieties. These natural products exhibit a broad spectrum of biological activity of demonstrable therapeutic value. For example, bleomycin A (Tomohisa et al.), a widely prescribed anticancer drug, effects the oxidative degradation of DNA and uses a bithiazole moiety to bind its target DNA sequences (Vanderwall et al., 1997). Bacitracin (Ming et al., 2002), a thiazoline-containing peptide antibiotic, interdicts bacterial cell wall new biosynthesis by complexation with C55-bactoprenolpyrophosphate. Thiangazole (Kunze et al., 1993) contains a tandem array of one oxazole and three thiazolines and exhibits antiviral activity (Jansen et al., 1992). Yet other oxazole/thiazole-containing natural products such as thiostrepton (Anderson et al., 1970) and GE2270A (Selva et al., 1997) inhibit translation steps in bacterial protein synthesis. More than 1000 alkaloids with the indole skeleton have been reported from microorganisms. One-third of these compounds are peptides with masses beyond 500 Da where the indole is tryptophan derived. The structural variety of the remaining two-thirds is higher, and their biological activity seems to cover a broader range, including antimicrobial, antiviral, cytotoxic, insecticidal, antithrombotic, or enzyme inhibitory activity.

### BRIEF SUMMARY

The present invention relates to an isolated strain of *Burkholderia* A396 (NRRL Accession No. B-50319) which has the following characteristics:
(A) a 16S rRNA gene sequence comprising the forward sequences having at least 99% identity to the sequences set forth in SEQ ID NO:8, 11, and 12 and reverse sequences having at least 99% identity to the sequences set forth in SEQ ID NO:9, 10, 13, 14 and 15;
(B) pesticidal activity;
(C) produces a pesticidal compound selected from
   (i) a compound having a structure 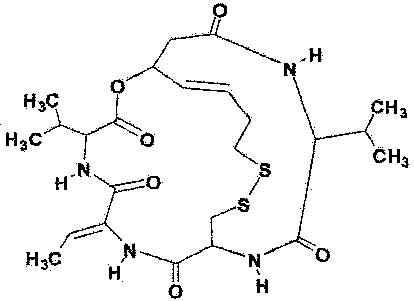
   (ii) a compound having a structure 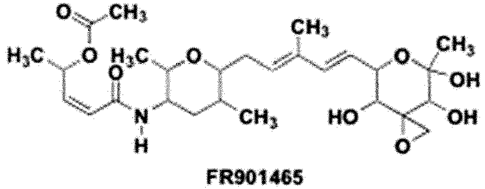
   (iii) a compound having a structure 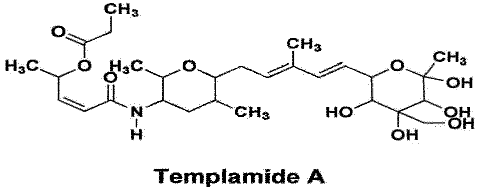
   (iv) a compound having a structure 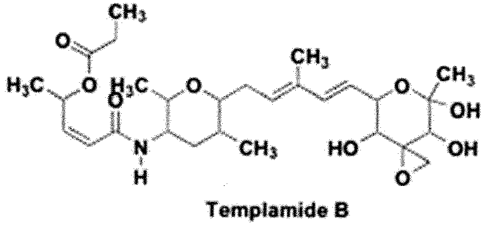
   (v) a compound having a structure 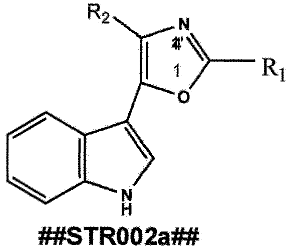 wherein R1 is isobutyl and R2 is carboxylic acid methyl ester; and
   (vi) a compound having a structure 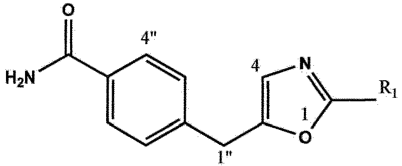 wherein R1 is isobutyl;
(D) is non-pathogenic to vertebrate animals; and
(E) is susceptible to kanamycin, chloramphenicol, ciprofloxacin, piperacillin, imipenem, and a combination of sulphamethoxazole and trimethoprim.

Disclosed herein are isolated compounds which are optionally obtainable or derived from *Burkholderia* species, or alternatively, organisms capable of producing these compounds that can be used to control various pests, particularly plant phytopathogenic pests, examples of which include but are not limited to insects, nematodes, bacteria, fungi. These compounds may also be used as herbicides.

In particular, the isolated pesticidal compounds of the present invention obtainable from a Burkholderia species are selected from
(i) a compound having a structure 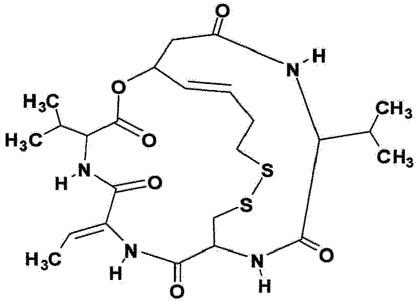
(ii) a compound having a structure 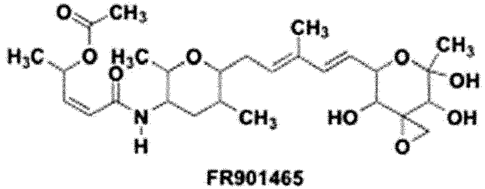
(iii) a compound having a structure 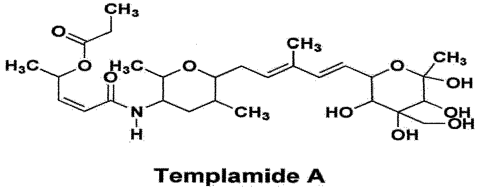
(iv) a compound having a structure 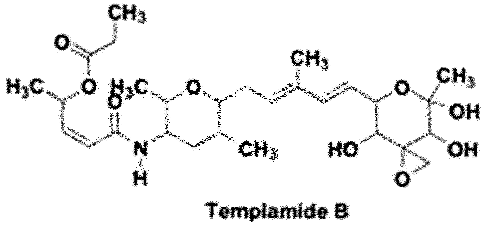
(v) a compound having a structure 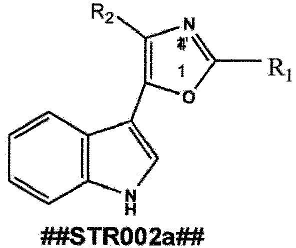 wherein R1 is isobutyl and R2 is carboxylic acid methyl ester; and
(vi) a compound having a structure 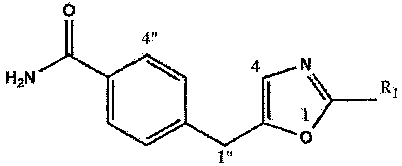 wherein R1 is isobutyl;

Also provided are methods of obtaining the compounds set forth above. In particular, the method comprises culturing the *Burkholderia* strain disclosed herein and producing the compound. Further provided is a method for isolating these compounds by isolating the compound(s) produced by a *Burkholderia* strain comprising isolating compounds produced from a supernatant of a culture of said *Burkholderia* strain.

Further provided is a combination comprising (a) a first substance selected from the group consisting of (i) a pure culture, cell fraction or supernatant derived from the *Burkholderia* strain set forth above or extract thereof for use optionally as a pesticide; (ii) one or more of the compounds set forth above (b) optionally a second substance, wherein said second substance is a chemical or biological pesticide and (c) optionally at least one of a carrier, diluent, surfactant, adjuvant, or pesticide. In a particular embodiment, the combination is a composition. In a related aspect, provided herein is a seed coated with said composition.

In a related aspect, disclosed is a method for modulating pest infestation in a plant comprising applying to the plant and/or seeds thereof and/or substrate used for growing said plant and/or a method for modulating emergence and/or growth of monocotyledonous, sedge or dicotyledonous weeds comprising applying to said weed or soil an amount of
(I) (a) the isolated compounds set forth above and (b) optionally another substance, wherein said substance is a pesticide (e.g. nematocide, herbicide, fungicide, insecticide) or
(II) the composition or combination set forth above
in an amount effective to modulate pest infestation and/or emergence or growth of monocotyledonous, sedge or dicotyledonous weeds.

In another related aspect, provided is the use of the strains, cultures, extracts, supernatants, combinations, compounds set forth above for modulating pest infestation in a plant comprising applying to the plant and/or seeds thereof and/or substrate used for growing said plant and/or a method for modulating emergence and/or growth of monocotyledonous, sedge or dicotyledonous weeds.

### BRIEF DESCRIPTION OF THE FIGURES

Figure 1 shows the comparison of the growth rate of *Burkholderia* A396 *to Burkholderia multivorans* ATCC 17616.
Figure 2 shows the effect of *Burkholderia* A396 extract on bindweed.

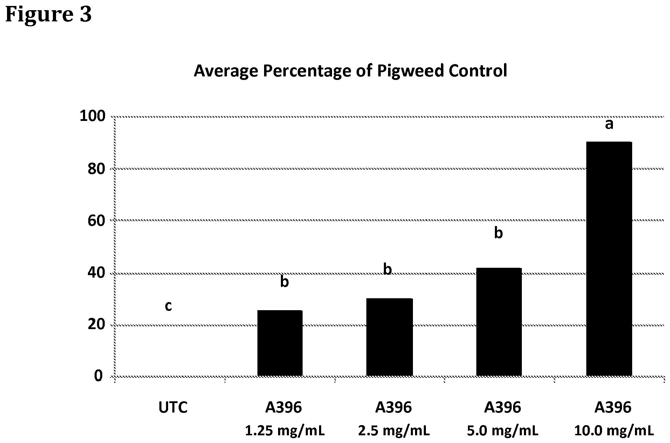

Figure 3 shows the effect of *Burkholderia* A396 extract on pigweed.
Figure 4 shows the effect of *Burkholderia* A396 extract on Cabbage looper (*Tricoplusia ni*)*.*
Figure 5 shows the effect of *Burkholderia* A396 culture broth on Beet armyworm (*Spodoptera exigua*)*.*
Figure 6 shows the effect of *Burkholderia* A396 culture broth on the motility of juvenile root-knot nematodes (*Meloidogyne incognita*).
Figure 7 is a schematic representation of purification scheme for obtaining the templazole and templamide compounds.
Figure 8 shows results of an *in vitro* assay to test the fungicidal effect of FR90128 on *Botrytis cinerea* (left) and *Phytophtora* sp. (right).
Figure 9 shows the effect of *Burkholderia* A396 culture broth on the average gall index (% control) of cucumber roots cv. Toschka inoculated with 3000 eggs of *Meloidogyne sp.* 14 days after inoculation and application.
Figure 10 Effect of *Burkholderia* A396 culture broth on the average gall index of cucumber roots cv. Toschka inoculated with 3000 eggs of *Meloidogyne sp.* 14 days after inoculation and application.

### DETAILED DESCRIPTION OF EMBODIMENTS

While the compositions and methods heretofore are susceptible to various modifications and alternative forms, exemplary embodiments will herein be described in detail.

Where a range of values is provided, it is understood that each intervening value, to the tenth of the unit of the lower limit unless the context clearly dictates otherwise, between the upper and lower limit of that range and any other stated or intervening value in that stated range, is included therein. Smaller ranges are also included. The upper and lower limits of these smaller ranges are also included therein, subject to any specifically excluded limit in the stated range.

Unless defined otherwise, all technical and scientific terms used herein have the same meaning as commonly understood by one of ordinary skill in the art to which this invention belongs.

It must be noted that as used herein and in the appended claims, the singular forms "a," "and" and "the" include plural references unless the context clearly dictates otherwise.

As defined herein, "derived from" means directly isolated or obtained from a particular source or alternatively having identifying characteristics of a substance or organism isolated or obtained from a particular source.

As defined herein, an "isolated compound" is free of other compounds or substances.

As used herein, the term "alkyl" refers to a monovalent straight or branched chain hydrocarbon group having from one to about 12 carbon atoms, including methyl, ethyl, n-propyl, isopropyl, n-butyl, isobutyl, tert-butyl, n-hexyl, and the like.

As used herein, "substituted alkyl" refers to alkyl groups further bearing one or more substituents selected from hydroxy, alkoxy, mercapto, cycloalkyl, substituted cycloalkyl, heterocyclic, substituted heterocyclic, aryl, substituted aryl, heteroaryl, substituted heteroaryl, aryloxy, substituted aryloxy, halogen, cyano, nitro, amino, amido, --C(O)H, acyl, oxyacyl, carboxyl, sulfonyl, sulfonamide, sulfuryl, and the like.

As used herein, "alkenyl" refers to straight or branched chain hydrocarbyl groups having one or more carbon-carbon double bonds, and having in the range of about 2 up to 12 carbon atoms, and "substituted alkenyl" refers to alkenyl groups further bearing one or more substituents as set forth above.

As used herein, "alkynyl" refers to straight or branched chain hydrocarbyl groups having at least one carbon-carbon triple bond, and having in the range of about 2 up to 12 carbon atoms, and "substituted alkynyl" refers to alkynyl groups further bearing one or more substituents as set forth above.

As used herein, "aryl" refers to aromatic groups having in the range of 6 up to 14 carbon atoms and "substituted aryl" refers to aryl groups further bearing one or more substituents as set forth above.

As used herein, "heteroaryl" refers to aromatic rings containing one or more heteroatoms (e.g., N, O, S, or the like) as part of the ring structure, and having in the range of 3 up to 14 carbon atoms and "substituted heteroaryl" refers toheteroaryl groups further bearing one or more substituents as set forth above.

As used herein, "alkoxy" refers to the moiety --O-alkyl-, wherein alkyl is as defined above, and "substituted alkoxy" refers to alkoxyl groups further bearing one or more substituents as set forth above.

As used herein, "thioalkyl" refers to the moiety --S-alkyl-, wherein alkyl is as defined above, and "substituted thioalkyl" refers to thioalkyl groups further bearing one or more substituents as set forth above.

As used herein, "cycloalkyl" refers to ring-containing alkyl groups containing in the range of about 3 up to 8 carbon atoms, and "substituted cycloalkyl" refers to cycloalkyl groups further bearing one or more substituents as set forth above.

As used herein, "heterocyclic", refers to cyclic (i.e., ring-containing) groups containing one or more heteroatoms (e.g., N, O, S, or the like) as part of the ring structure, and having in the range of 3 up to 14 carbon atoms and "substituted heterocyclic" refers to heterocyclic groups further bearing one or more substituent's as set forth above.

### The Burkholderia Strain

The *Burkholderia* strain set forth herein is a *non-Burkholderia cepacia* complex, *non-Burkholderia plantari, non-Burkholderia gladioli, Burkholderia sp* and non-pathogenic to vertebrates, such as birds, mammals and fish. This strain may be isolated from a soil sample using procedures known in the art and described by Lorch et al., 1995. The *Burkholderia* strain may be isolated from many different types of soil or growth medium. The sample is then plated on potato dextrose agar (PDA). The bacteria are gram negative, and it forms round, opaque cream-colored colonies that change to pink and pinkish-brown in color and mucoid or slimy over time.

Colonies are isolated from the potato dextrose agar plates and screened for those that have biological, genetic, biochemical and/or enzymatic characteristics of the *Burkholderia* strain of the present invention set forth in the Examples below. In particular, the *Burkholderia* strain has a 16S rRNA gene comprising a forward sequence that is at least about 99.0%, preferably about 99.5%, more preferably about 99.9% and most preferably about 100% identical to the sequence set forth in SEQ ID NO: 8, 11 and 12 and a forward sequence that is at least about 99.0%, preferably about 99.5%, more preferably about 99.9% and most preferably about 100% identical to the sequence set forth in SEQ ID NO: 9, 10, 13, 14 and 15 as determined by clustal analysis. Furthermore, as set forth below, this *Burkholderia* strain may, as set forth below, have pesticidal activity, particularly, virucidal, herbicidal, germicidal, fungicidal, nematicidal, bactericidal and insecticidal and more particularly, herbicidal, insecticidal, fungicidal and nematicidal activity. It is not pathogenic to vertebrate animals, such as mammals, birds, and fish.

Additionally, the *Burkholderia* strain produces at least the pesticidal compounds set forth in the instant disclosure.

The *Burkholderia* strain is susceptible to kanamycin, chloramphenicol, ciprofloxacin, piperacillin , imipenem, and a combination of sulphamethoxazole and trimethoprim and contains the fatty acids 16:0, cyclo 17:0, 16:0 3- OH, 14:0, cyclo 19:0, 18:0.

This *Burkholderia* strain may be obtained by culturing a microorganism having the identifying characteristics of *"Burkholderia* A396 (NRRL Accession No. B-50319) on Potato Dextrose Agar (PDA) or in a fermentation medium containing defined carbon sources such as glucose, maltose, fructose, galactose, and undefined nitrogen sources such as peptone, tryptone, soytone, and NZ amine.

### Pesticidal Compounds

The pesticidal compound disclosed herein may have the following properties: (a) is obtainable from a novel *"Burkholderia* species, e.g., A396.

The present invention discloses an isolated compound having pesticidal activity obtainable from a Burkholderia species selected from
(i) a compound having a structure 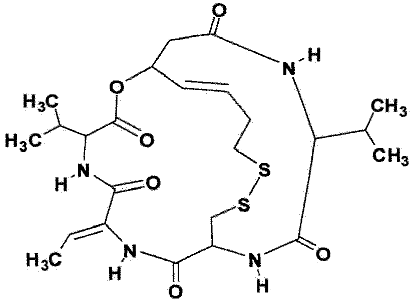
(ii) a compound having a structure 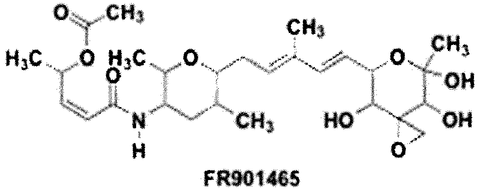
(iii) a compound having a structure 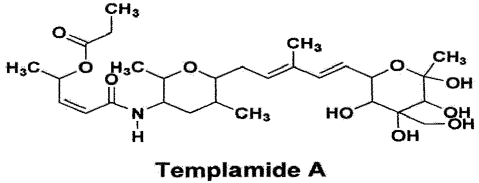
(iv) a compound having a structure 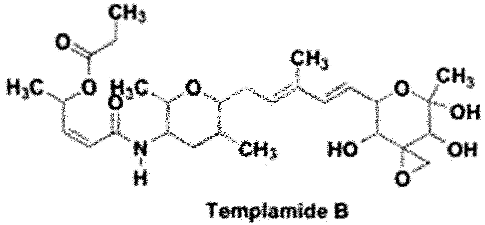
(v) a compound having a structure 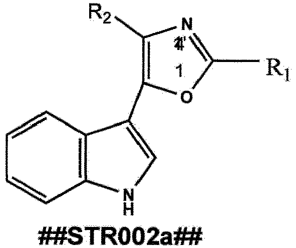 wherein R1 is isobutyl and R2 is carboxylic acid methyl ester; and
(vi) a compound having a structure 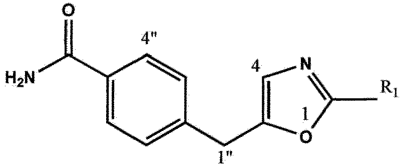 wherein R1 is isobutyl; 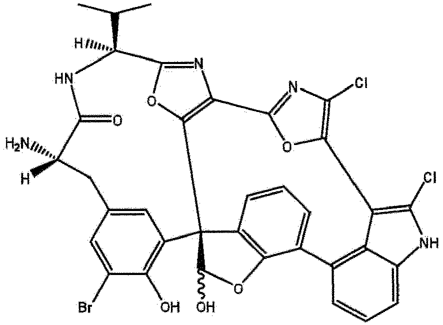
(xix)

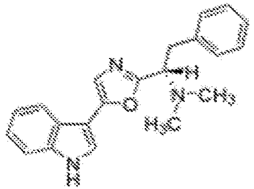

These are from either natural materials or compounds obtained from commercial sources or by chemical synthesis.

In a more particular embodiment, the compound is Templamide A with the following structure:

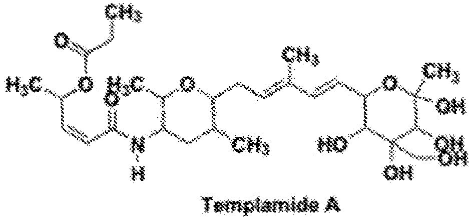

In a particular embodiment, the compound has the structure:

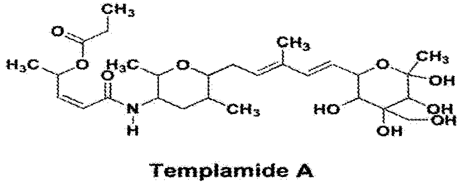

In a more particular embodiment, the compound is Templamide B with the following structure:

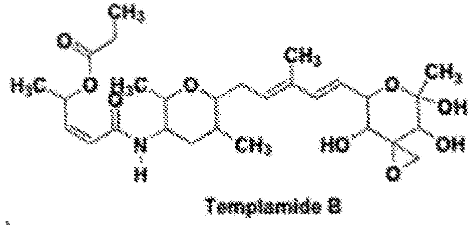

In a more particular embodiment, the compound is a known compound FR901465 which was isolated earlier from culture broth of a bacterium of *Pseudomonas* sp. No. 2663 (Nakajima et al. 1996) and had been reported to have anticancer activity with the following structure:

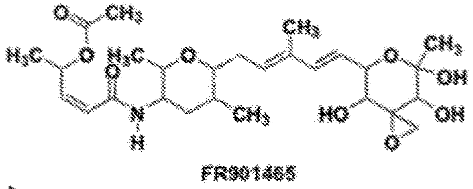

### Compositions

A substantially pure culture, cell fraction or supernatant and compounds produced by the *Burkholderia* strain of the present invention, may be formulated into pesticidal compositions.

The substances set forth above can be formulated in any manner. Non-limiting formulation examples include but are not limited to emulsifiable concentrates (EC), wettable powders (WP), soluble liquids (SL), aerosols, ultra-low volume concentrate solutions (ULV), soluble powders (SP), microencapsulation, water dispersed granules, flowables (FL), microemulsions (ME), nano-emulsions (NE), etc. In particular, the concentrate, powders, granules and emulsions may be freeze-dried. In any formulation described herein, percent of the active ingredient is within a range of 0.01% to 99.99%.

The compositions may be in the form of a liquid, gel or solid. Liquid compositions comprise pesticidal compounds derived from said *Burkholderia* strain, e.g. a strain having the identifying characteristics of *Burkholderia* A396 (NRRL Accession No. B-50319).

A solid composition can be prepared by suspending a solid carrier in a solution of pesticidal compounds and drying the suspension under mild conditions, such as evaporation at room temperature or vacuum evaporation at 65°C or lower.

A composition of the invention may comprise gel-encapsulated compounds derived from the *Burkholderia* strain of the present invention. Such gel-encapsulated materials can be prepared by mixing a gel-forming agent (e.g., gelatin, cellulose, or lignin) with a solution of pesticidal compounds used in the method of the invention; and inducing gel formation of the agent.

The composition may additionally comprise a surfactant to be used for the purpose of emulsification, dispersion, wetting, spreading, integration, disintegration control, stabilization of active ingredients, and improvement of fluidity or rust inhibition. In a particular embodiment, the surfactant is a non-phytotoxic non-ionic surfactant which preferably belongs to EPA List 4B. In another particular embodiment, the nonionic surfactant is polyoxyethylene (20) monolaurate. The concentration of surfactants may range between 0.1-35% of the total formulation, preferred range is 5-25%. The choice of dispersing and emulsifying agents, such as non-ionic, anionic, amphoteric and cationic dispersing and emulsifying agents, and the amount employed is determined by the nature of the composition and the ability of the agent to facilitate the dispersion of these compositions.

The composition may further comprise another microorganism and/or pesticide (e.g, nematocide, fungicide, insecticide). The microorganism may include but is not limited to an agent derived from *Bacillus* sp., *Pseudomonas* sp., *Brevabacillus sp., Lecanicillium* sp., non*-Ampelomyces* sp., *Pseudozyma* sp., *Streptomyces* sp, *Burkholderia* sp, *Trichoderma* sp, *Gliocladium* sp. Alternatively, the agent may be a natural oil or oil-product having fungicidal and/or insecticidal activity (e.g., paraffinic oil, tea tree oil, lemongrass oil, clove oil, cinnamon oil, citrus oil, rosemary oil).

The composition, in particular, may further comprise an insecticide. The insecticide may include but is not limited to avermectin, *bacillus thuringiensis*, neem oil and azadiractin, spinosads, *Chromobacterium subtsugae,* eucalyptus extract, entomopathogenic bacterium or fungi such a *Beauveria bassiana, and Metarrhizium anisopliae* and chemical insecticides including but not limited to organochlorine compounds, organophosphorous compounds, carbamates, pyrethroids, and neonicotinoids.

The composition my further comprise a nematicide. The nematicide may include, but is not limited to chemical nematicides such as fenamiphos, aldicarb, oxamyl, carbofuran, natural product neamticide, avermectin, the fungi *Paecilomyces lilacinas and Muscodor spp.,* the bacteria *Bacillus firmus and other Bacillus spp.* and *Pasteuria penetrans.*

The composition may further comprise a biofungicide such as extract of *R. sachalinensis* (Regalia) or a fungicide. Such fungicides include, but are not limited to, a single site anti-fungal agent which may include but is not limited to benzimidazole, a demethylation inhibitor (DMI) (e.g., imidazole, piperazine, pyrimidine, triazole), morpholine, hydroxypyrimidine, anilinopyrimidine, phosphorothiolate, quinone outside inhibitor, quinoline, dicarboximide, carboximide, phenylamide, anilinopyrimidine, phenylpyrrole, aromatic hydrocarbon, cinnamic acid, hydroxyanilide, antibiotic, polyoxin, acylamine, phthalimide, benzenoid (xylylalanine). In yet a further embodiment, the antifungal agent is a demethylation inhibitor selected from the group consisting of imidazole (e.g., triflumizole), piperazine, pyrimidine and triazole (e.g.,bitertanol, myclobutanil, penconazole, propiconazole, triadimefon, bromuconazole, cyproconazole, diniconazole, fenbuconazole, hexaconazole, tebuconazole, tetraconazole, propiconazole).

The antimicrobial agent may also be a multi-site non-inorganic, chemical fungicide selected from the group consisting of a nitrile (e.g., chloronitrile or fludioxonil), quinoxaline, sulphamide, phosphonate, phosphite, dithiocarbamate, chloralkythios, phenylpyridin-amine, cyano-acetamide oxime.

The compositions may be applied using methods known in the art. Specifically, these compositions may be applied to plants or plant parts. Plants are to be understood as meaning in the present context all plants and plant populations such as desired and undesired wild plants or crop plants (including naturally occurring crop plants). Crop plants can be plants which can be obtained by conventional plant breeding and optimization methods or by biotechnological and genetic engineering methods or by combinations of these methods, including the transgenic plants and including the plant cultivars protectable or not protectable by plant breeders' rights. Plant parts are to be understood as meaning all parts and organs of plants above and below the ground, such as shoot, leaf, flower and root, examples which may be mentioned being leaves, needles, stalks, stems, flowers, fruit bodies, fruits, seeds, roots, tubers and rhizomes. The plant parts also include harvested material, and vegetative and generative propagation material, for example cuttings, tubers, rhizomes, offshoots and seeds.

Treatment of the plants and plant parts with the compositions set forth above may be carried out directly or by allowing the compositions to act on their surroundings, habitat or storage space by, for example, immersion, spraying, evaporation, fogging, scattering, painting on, injecting. In the case that the composition is applied to a seed, the composition may be applied to the seed as one or more coats prior to planting the seed using one or more coats using methods known in the art.

As noted above, the compositions may be herbicidal compositions. The composition may further comprise one or more herbicides. These may include, but are not limited to, a bioherbicide and/or a chemical herbicide. The bioherbicide may be selected from the group consisting of clove, cinnamon, lemongrass, citrus oils, orange peel oil, tentoxin, cornexistin, AAL-toxin, leptospermone, thaxtomin, sarmentine, momilactone B, sorgoleone, ascaulatoxin and ascaulatoxin aglycone. The chemical herbicide may include, but is not limited to, diflufenzopyr and salts thereof, dicamba and salts thereof, topramezone, tembotrione, S-metolachlor, atrazine, mesotrione, primisulfuron-methyl, 2,4-dichlorophenoxyacetic acid, nicosulfuron, thifensulfuron-methyl, asulam, metribuzin, diclofop-methyl, fluazifop, fenoxaprop-p-ethyl, asulam, oxyfluorfen, rimsulfuron, mecoprop, and quinclorac, thiobencarb, clomazone, cyhalofop, propanil, bensulfuron-methyl, penoxsulam, triclopyr, imazethapyr, halosulfuron-methyl, pendimethalin, bispyribac-sodium, carfentrazone ethyl, sodium bentazon/sodium acifluorfen, glyphosate, glufosinate and orthosulfamuron.

Herbicidal compositions may be applied in liquid or solid form as pre-emergence or post-emergence formulations.

For pre-emergence dry formulations, the granule size of the carrier is typically 1-2 mm (diameter) but the granules can be either smaller or larger depending on the required ground coverage. Granules may comprise porous or non-porous particles.

For post-emergence formulations, the formulation components used may contain smectite clays, attapulgite clays and similar swelling clays, thickeners such as xanthan gums, gum Arabic and other polysaccharide thickeners as well as dispersion stabilizers such as nonionic surfactants (for example polyoxyethylene (20) monolaurate).

### Uses

The compositions and pesticidal compounds derived from the *Burkholderia* strain set forth herein may be used as pesticides, particularly as insecticides, nematocides, fungicides and herbicides.

Specifically, nematodes that may be controlled using the method set forth above include but are not limited to parasitic nematodes such as root-knot, ring, sting, lance, cyst, and lesion nematodes, including but not limited to *Meloidogyne, Heterodera* and *Globodera* spp; particularly *Meloidogyne incognita* (root knot nematodes), as well as *Globodera rostochiensis* and *globodera pailida* (potato cyst nematodes); *Heterodera glycines* (soybean cyst nematode); *Heterodera schachtii* (beet cyst nematode); and *Heterodera avenae* (cereal cyst nematode).

Phytopathogenic insects controlled by the method of the present invention include but are not limited to insects from the order
(a) Lepidoptera, for example, *Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni and Yponomeuta spp.;*
*(b) Coleoptera,* for example, *Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp-, Scarabeidae, Sitophilus spp., Sitotroga spp., Tenebrio spp., Tribolium spp. and Trogoderma spp.; (c) Orthoptera, for example, Blatta spp., Blattella spp., Gryllotalpa spp., Leucophaea maderae, Locusta spp., Periplaneta spp. and Schistocerca spp.; (d) Isoptera, for example, Reticulitermes spp.; (e) Psocoptera, for example, Liposcelis spp.; (f) Anoplura, for example, Haematopinus spp., Linognathus spp., Pediculus spp., Pemphigus spp. and Phylloxera spp.; (g) Mallophaga, for example, Damalinea spp. and Trichodectes spp.; (h) Thysanoptera, for example, Frankliniella spp., Hercinotnrips spp., Taeniothrips spp., Thrips palmi, Thrips tabaci and Scirtothrips aurantii; (i) Heteroptera, for example, Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. and Tniatoma spp.; (j) Homoptera, for example, Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae and Unaspis citri; (k) Hymenoptera, for example, Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. and Vespa spp.; (l) Diptera, for example, Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. and Tipula spp.; (m) Siphonaptera, for example, Ceratophyllus spp. und Xenopsylla cheopis* and (n) from the order Thysanura, for example, *Lepisma saccharina.* The active ingredients according to the invention may further be used for controlling crucifer flea beetles (*Phyllotreta* spp.), root maggots (*Delia* spp.), cabbage seedpod weevil (*Ceutorhynchus* spp.) and aphids in oil seed crops such as canola (rape), mustard seed, and hybrids thereof, and also rice and maize.

In a particular embodiment, the insect may be a member of the *Spodoptera,* more particularly, *Spodoptera exigua, Myzus persicae, Plutella xylostella* or *Euschistus* sp.

The substances and compositions may also be used to modulate emergence in either a pre-emergent or post-emergent formulation of monocotyledonous, sedge or dicotyledonous weeds. In a particular embodiment, the weeds may be *Chenopodium album, Abutilon theophrasti, Helianthus annuus, Ambrosia artemesifolia, Amaranthus retroflexus, Convolvulus arvensis, Brassica kaber, Taraxacum officinale, Solanum nigrum, Malva neglect,, Setaria lutescens, Bromus tectorum, Poa annua, Poa pratensis, Lolium perenne* L. var. Pace, *Festuca arundinaceae Schreb.* var. Aztec II, Anthem II, LS 1100, *Echinochloa crus-galli, Lactuca sativa.* The *Burkholderia* strain, compounds and compositions set forth above may also be used as a fungicide. The targeted fungus may be a *Fusarium* sp., *Botrytis* sp., *Monilinia* sp., *Colletotrichum sp, Verticillium sp.; Microphomina* sp., *Phytophtora sp, Mucor* sp., *Podosphaera sp. Rhizoctonia sp., Peronospora sp., Geotrichum sp., Phoma, and Penicillium.* In another most particular embodiment, the bacteria are *Xanthomonas.*

The invention will now be described in greater detail by reference to the following non-limiting examples.

### EXAMPLES

The compositions and methods set forth above will be further illustrated in the following, non-limiting Examples. The examples are illustrative of various embodiments only and do not limit the claimed invention regarding the materials, conditions, weight ratios, process parameters and the like recited herein.

### 1. Example 1. Isolation and identification of the microbe

### 1.1 Isolation of the microorganism

The microbe is isolated using established techniques know to the art from a soil sample collected under an evergreen tree at the Rinnoji Temple, Nikko, Japan. The isolation is done using potato dextrose agar (PDA) using a procedure described in detail by Lorch et al. , 1995. In this procedure, the soil sample is first diluted in sterile water, after which it is plated in a solid agar medium such as potato dextrose agar (PDA). The plates are grown at 25°C for five days, after which individual microbial colonies are isolated into separate PDA plates. The isolated bacterium is gram negative, and it forms round, opaque cream-colored colonies that change to pink and pinkish-brown in color and mucoid or slimy over time.

### 1.2. Identification on the microorganism

The microbe is identified based on gene sequencing using universal bacterial primers to amplify the 16S rRNA region. The following protocol is used: *Burkholderia sp* A396 is cultured on potato-dextrose agar plates. Growth from a 24 hour-old plate is scraped with a sterile loop and re-suspended in DNA extraction buffer. DNA is extracted using the MoBio Ultra Clean Microbial DNA extraction kit. DNA extract is checked for quality/quantity by running *5µ*l on a 1% agarose gel.

PCR reactions are set up as follows: 2 *µ*l DNA extract, 5 *µ*l PCR buffer, 1 *µ*l dNTPs (10 mM each), 1.25 *µ*l forward primer (27F; 5'-AGAGTTTGATCCTGGCTCAG-3' (SEQ ID NO:1), 1.25 *µ*l reverse primer (907R; 5'-CCGTCAATTCCTTTGAGTTT-3' (SEQ ID NO:2)) and 0.25 *µ*l Taq enzyme. The reaction volume is made up to 50 *µ*l using sterile nuclease-free water. The PCR reaction includes an initial denaturation step at 95°C for 10 minutes, followed by 30 cycles of 94°C/30 sec, 57°C/20 sec, 72°C/30 sec, and a final extension step at 72°C for 10 minutes.

The product's approximate concentration and size is calculated by running a 5 *µ*l volume on a 1% agarose gel and comparing the product band to a mass ladder.

Excess primers, dNTPs and enzyme are removed from the PCR product with the MoBio PCR clean up kit. The cleaned PCR product as directly sequenced using primers 27F (same as above), 530F (5'-GTGCCAGCCGCCGCGG-3' (SEQ ID NO:3)), 1114F (5'-GCAACGAGCGCAACCC (SEQ ID NO:4)) and 1525R (5'-AAGGAGGTGWTCCARCC-3' (SEQ ID NO:5)), 1100R (5'-GGGTTGCGCTCGTTG-3' (SEQ ID NO:6)), 519R (5'-GWATTACCGCGGCKGCTG-3' (SEQ ID NO:7).

The 16S rRNA gene sequence of strain A396 is compared with the available 16s rRNA gene sequences of representatives of the β-proteobacteria using BLAST. Strain A395 A396 is closely related to members of the *Burkholderia cepacia* complex, with 99% or higher similarity to several isolates of *Burkholderia multivorans, Burkholderia vietnamensis,* and *Burkholderia cepacia.* A BLAST search excluding the *B. cepacia* complex, showed 98% similarity to *B. plantarii, B. gladioli* and *Burkholderia sp.* isolates.

A distance tree of results using the neighbor joining method, showed that A396 is related to *Burkholderia multivorans* and other *Burkholderia cepacia* complex isolates. *Burkholderia plantarii* and *Burkholderia glumae* grouped in a separate branch of the tree.

The isolated *Burkholderia* strain was found to contain the following sequences: forward sequence, DNA sequence with 27F primer, 815 nucleotides (SEQ ID NO:8); reverse sequence, 1453 bp, using primers 1525R, 1100R, 519R (SEQ ID NO:9); reverse sequence 824 bp using primer 907R (SEQ NO: 10); forward sequence 1152 bp using primer 530F (SEQ ID NO:11); forward sequence 1067 bp using 1114F primer (SEQ ID NO:12); reverse sequence 1223 bp using 1525R primer (SEQ NO:13); reverse sequence 1216 bp using 1100R primer (SEQ ID NO:14); reverse sequence 1194 bp using 519R primer (SEQ ID NO:15).

### 1.3. Proof that Burkholderia A396 does not belong to Burkholderia cepacia complex

### 1.3.1 Molecular Biology work using specific PCR primers

In order to confirm the identification of *Burkholderia A396* as *Burkholderia multivorans,* additional sequencing of housekeeping genes is performed. *Burkholderia multivorans* is a known member of the *Burkholderia cepacia* complex. Efforts are focused on PCR of recA genes, as described by Mahenthiralingam et al., 2000. The following primers are used: (a) BCR1 and BCR2 set forth in Mahenthiralingam et al., 2000 to confirm *B. cepacia* complex match and (b) BCRBM1 and BCRBM2 set forth Mahenthiralingam et al, 2000 to confirm *B. multivorans* match. A product-yielding PCR reaction for the first primer set would confirm that the microbe belongs to the *B. cepacia* complex. A product-yielding PCR reaction for the second primer set would confirm that the microbe is indeed B. *multivorans.*

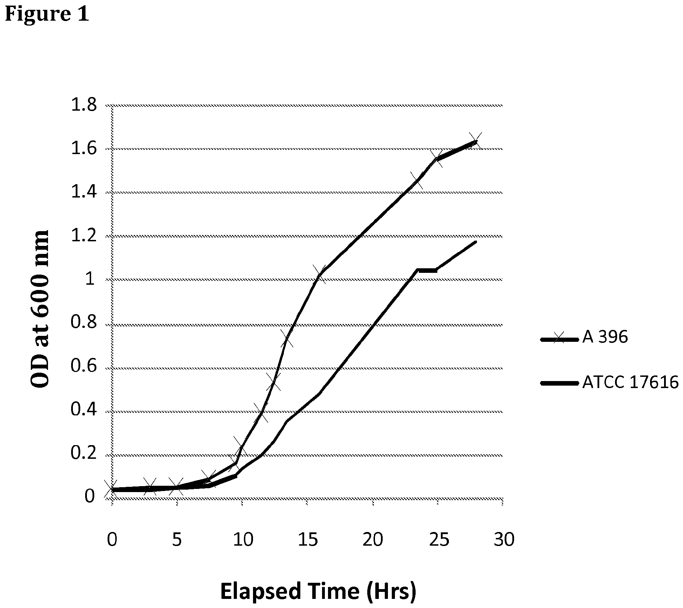

No PCR product is obtained for either pair of primers. The performance of the PCR reaction and primers is tested using *Burkholderia multivorans* ATCC 17616 (positive control) and *Pseudomonas fluorescens* (negative control). Strong bands are observed both for *B. multivorans* using both sets of primers. No bands are observed for *Pseudomonas fluorescens.* The results indicate that A396 is a Burkholderia, but not a member of the *B. cepacia* complex, and not *Burkholderia multivorans.* This is also demonstrated in a comparative culture experiment in which both A396 and a type culture of *B. multivorans* are grown side-by-side in a shake culture, and the growth is monitored daily using optical density measurements at 600 nm. Under the set conditions, the novel species A396 grew much faster than the *B. multivorans* type strain (Figure 1).

### 1.3.2 DNA-DNA Hybridization

In order to confirm that isolate A396 is a new species of *Burkholderia,* a DNA-DNA hybridization experiment with *Burkholderia multivorans* (the closest 16S rRNA sequence match) is conducted. Biomass for both A396 and *B. multivorans* is produced in ISP2 broth, grown over 48 hours at 200 rpm/25°C in Fernbach flasks. The biomass is aseptically harvested by centrifugation. The broth is decanted and the cell pellet is resuspended in a 1:1 solution of water: isopropanol. DNA-DNA hybridization experiments are performed by the DSMZ, the German Collection of Microorganisms and Cell Cultures in Germany. DNA is isolated using a French pressure cell (Thermo Spectronic) and is purified by chromatography on hydroxyapatite as described by Cashion et al., 1977. DNA-DNA hybridization is carried out as described by De Ley et al., 1970 under consideration of the modifications described by Huss et al., 1983 using a model Cary 100 Bio UV/VIS-spectrophotometer equipped with a Peltier thermostatted 6x6 multicell changer and a temperature controller with *in-situ* temperature probe (Varian). DSMZ reported % DNA-DNA similarly between A396 and *Burkholderia multivorans* of 37.4%. The results indicate that *Burkholderia sp* strain A396 does not belong to the species *Burkholderia multivorans* when the recommendations of a threshold value of 70% DNA-DNA similarity for the definition of bacterial species by the ad hoc committee (Wayne et al., 1987) are considered.

### 1.4. Biochemical profile using Biolog GN2 plates

For the carbon source utilization profile, A396 is grown overnight on Potato Dextrose Agar (PDA). The culture is transferred to BUG agar to produce an adequate culture for Biolog experiments as recommended by the manufacturer (Biolog, Hayward, CA).

The biochemical profile of the microorganism is determined by inoculating onto a Biolog GN2 plate and reading the plate after a 24-hour incubation using the MicroLog 4-automated microstation system. Identification of the unknown bacteria is attempted by comparing its carbon utilization pattern with the Microlog 4 Gram negative database.

No clear definitive matches are found to the Biolog profile. The closest matches all had less than 35% similarity with A396: *Pseudomonas spinosa (Burkholderia), Burkholderia cepacia, and Burkholderia pseudomallei.* The results are shown in Table I.

**Table 1. Biochemical Profile of A396**

| **Substrate** | **Result** | **Substrate** | **Result** |
|---|---|---|---|
| Cyclodextrin | - | L-arabinose | - |
| Dextrin | - | D-arabitol | - |
| Glycogen | - | D-cellobiose | - |
| Tween 40 | + | Erythritol | - |
| Tween 80 | + | D-Fructose | - |
| N-acetyl-D-Galactoseamine | - | L-Fucose | - |
| N-acetyl-D-glucosamine | - | D-Galactose | +/- |
| Adonitol | - | Gentibiose | - |
| Succinic Acid Mon-methyl ester | - | D-Glucose | + |
| Acetic acid | - | m-Inositol | - |
| Cis-aconitic acid | - | D-Lactose | - |
| Citric acid | - | Lactulose | - |
| Formic acid | + | Maltose | - |
| D-Galactonic Acid Lactone | - | D-Mannitol | - |
| D-Galacturonic Acid | - | D-Mannose | - |
| D-Gluconic acid | - | D-Melibiose | - |
| D-Glucosaminic acid | - | β-methyl-D-glucoside | - |
| D-Glucuronic Acid | - | D-Psicose | - |
| α-hydroxyburytic acid | - | D-Raffinose | - |
| β-hydroxybutyric acid | + | L-Rhamonose | - |
| γ-hydroxybutyric acid | - | D-Sorbitol | - |
| p-hydroxyphenylacetic acid | - | Sucrose | - |
| Itaconic acid | - | D-Trehalose | + |
| α-keto butyric acid | - | Turanose | - |
| α-keto glutaric acid | - | Xylitol | - |
| α-ket valeric acid | - | Pyruvic Acid Methyl esther | - |
| D,L-Lactic acid | - | Uridine | - |
| Malonic acid | - | Thymidine | - |
| Propionic acid | + | Phenyethyl-amine | - |
| Quinic acid | - | Putrescine | - |
| D-Saccharic acid | - | 2-aminoethanol | - |
| Sebacic acid | - | 2,3-Butanediol | - |
| Succinic Acid | + | Glycerol | +/- |
| Bromosuccinic acid | - | D,L-a-glycerol phosphate | +/- |
| Succinamic acid | - | α-D-Glucose-1-phosphate | - |
| Glucuronamide | - | D-glucose-6-phosphate | + |
| L-alaninamide | + | γ-amino butyric acid | + |
| D-Alanine | - | Urocanic acid | - |
| L-alanine | + | Inosine | - |
| L-alanyl-glycine | - | L-phenylalanine | + |
| L-asparagine | + | L-proline | - |
| L-aspartic acid | +/- | L-pyroglutamic acid | - |
| L-glutamic acid | + | D-serine | - |
| Glycyl-L-Aspartic acid | - | L-serine | - |
| Glycyl-L-glutamic acid | - | L-threonine | - |
| L-histidine | - | D,L-carnitine | - |
| Hydroxy-L-proline | + | L-ornithine | - |
| L-leucine | - | | |

### 1.5. Fatty acid composition

After incubation for 24 hours at 28°C, a loopful of well-grown cells are harvested and fatty acid methyl esters are prepared, separated and identified using the Sherlock Microbial Identification System (MIDI) as described (see Vandamme et al., 1992). The predominant fatty acids present in the *Burkholderia* A396 are as follows: 16:0 (24.4%), cyclo 17:0 (7.1%), 16:0 3- OH (4.4%), 14:0 (3.6%), 19:0 ω8*c* (2.6%) cyclo, 18:0 (1.0%). Summed feature 8 (comprising 18:1 ω7*c*) and summed feature 3 (comprising of 16:1 ω7c and 16:1 ω6*c*) corresponded to 26.2% and 20.2 % of the total peak area, respectively. Summed feature 2 comprising 12:0 ALDE, 16:1 iso I, and 14:0 3-OH) corresponded to 5.8% of the total peak area while summed feature 5 comprising 18:0 ANTE and 18:2 ω6,9c corresponded to 0.4%. Other fatty acids detected in A396 in minor quantities included: 13:1 at 12-13 (0.2%), 14:1 ω5c (0.2%), 15:0 3-OH (0.13%), 17:1 ω7c (0.14%), 17:0 (0.15%), 16:0 iso 3-OH (0.2%), 16:0 2-OH (0.8%), 18:1 ω7c 11-methyl (0.15%), and 18:1 2-OH (0.4%).

A comparison of the fatty acid composition of A396 with those of known microbial strains in the MIDI database suggested that the fatty acids in the novel strain A396 were most similar with those of *Burkholderia cenocepacia.*

### 1.6 Resistance to Antibiotics

Antibiotic susceptibility of *Burkholderia A396* is tested using antibiotic disks on Muller-Hinton medium as described in PML Microbiological's technical data sheet #535. Results obtained after 72-hour incubation at 25°C are presented in Table 2 below.

**Table 2: Susceptibility of MBI-206 to various antibiotics. +++ very susceptible, ++ susceptible, - resistant**

| | **Concentration (ug)** | **Susceptible** |
|---|---|---|
| Tetracycline | 30 | - |
| Kanamycin | 30 | +++ |
| Erythromycin | 15 | - |
| Streptomycin | 10 | - |
| Penicillin | 10 | - |
| Ampicillin | 10 | - |
| Oxytetracycline | 30 | - |
| Chloramphenicol | 30 | ++ |
| Ciprofloxacin | 5 | ++ |
| Gentamicin | 10 | - |
| Piperacillin | 100 | +++ |
| Cefuroxime | 30 | - |
| Imipenem | 10 | +++ |
| Sulphamethoxazole- | | - |
| Trimethoprim | 23.75/25 | ++ |

The results indicate that the antibiotic susceptibility spectrum of *Burkholderia A396* is quite different from pathogenic *B. cepacia* complex strains. *Burkholderia A396* is susceptible to kanamycin, chloramphenicol, ciprofloxacin, piperacillin, imipenem, and a combination of sulphamethoxazole and trimethoprim. As a comparison, Zhou et al., 2007 tested the susceptibility of 2,621 different strains in *B. cepacia complex* isolated from cystic fibrosis patients, and found that only 7% and 5% of all strains were susceptible to imipenem or ciprofloxacin, respectively. They also found 85% of all strains to be resistant to chloramphenicol (15% susceptible), and 95% to be resistant (5% susceptible) to the combination of sulphamethoxazole and trimethoprim. Results of Zhou et al., 2007 are similar to those of Pitt et al., 1996 who determined antibiotic resistance among 366 *B. cepacia* isolates and reported that most of them are resistant to ciprofloxacin, cefuroxime, imipenem, chloramphenicol, tetracycline, and sulphametoxacole.

### 2. Example 2. Burkholderia sp. as an Herbicide

### 2.1 Study #1

To confirm the activity found in the initial herbicide screen, an *in vivo* study is conducted using the Amberlite 7 XAD resin extract derived from a 5-day old whole cell broth of the novel *Burkholderia* species. The dried crude extract is resuspended in 4% ethanol and 0.2 % non-ionic surfactant (glycosperse) at a concentration of 10 mg/mL, and further diluted to a concentration of 5.0 mg/mL. The two samples are sprayed on 4-week old plants of bindweed (*Convolvulus arvensis*), and the plants are kept under growth lights at 25°C for 2 weeks, at which point, the phytotoxicity evaluations are performed. In the same study, 2-week old redroot pigweed plants are sprayed with increasing concentrations of the crude extract derived from the bacterial culture. The test concentrations are 1.25, 2.5, 5.0 and 10.0 mg/mL, and the plants are incubated as described above before phytotoxicity evaluations.

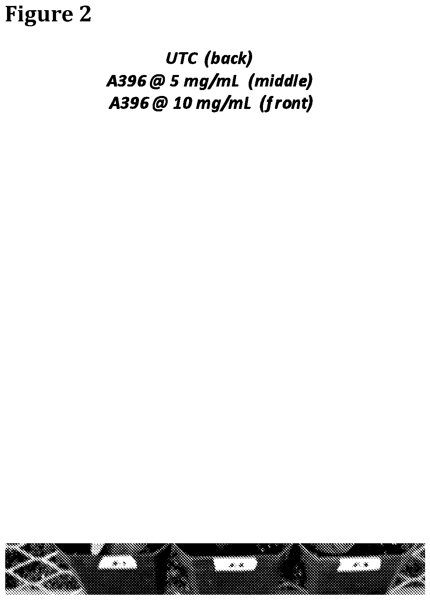

Results presented in Figures 2 (bindweed) and 3 (pigweed) show the phytotoxic effect of Burkholderia crude extract at different concentrations, and they show good herbicidal effect on pigweed even at low treatment concentrations. Both extract treatments (5 and 10 mg/mL) result in stunting on bindweed.

### 2.2 Study #2

A novel strain of *Burkholderia sp.* A396 is grown in an undefined mineral medium for 5 days (25°C, 200 rpm). The whole cell broth is extracted using XAD7 resin. The dried crude extract is resuspended in 4% ethanol and 0.2 % non-ionic surfactant at a concentration of 10 mg/mL, and further diluted to concentrations of 5.0, 2.5, and 1.25 mg/mL. All four test solutions are then tested on the following broadleaf and grass weed species listed in Table 3:

**Table 3. Broadleaf and Grass Weed Species Tested**

| **Common Name** | **Scientific Name** |
|---|---|
| Lambsquarter | *Chenopodium album* |
| Horseweed | *Conyza canadensis* |
| Curlydock | *Rumex crispus* |
| Crabgrass | *Digitaria sanguinalis* |
| Bluegrass | *Poa annua* |
| Dandelion | *Taraxacum officinale* |
| Nightshade | *Solanum nigrum* |
| Mustard | *Brassica kaber* |
| Mallow | *Malva neglecta* |
| Cocklebur | *Xanthium pensylvanicum* |
| Bermuda Grass | *Cynodon dactylon* |
| Foxtail | *Setaria lutescens* |
| Sowthistle | *Sonchus oleraceus* |

A solution of 0.2 % glycosperse and Roundup at 6 fl oz per gallon rate is used as negative and positive controls, respectively.

All plant species are tested in 4"x4" plastic pots in three replicates. The untreated control plants are sprayed with the carrier solution (4% Ethanol, 0.2% glycosperse) and the positive control plants with Roundup at a rate corresponding to 6 fl. oz/acre. Treated plants are kept in a greenhouse under 12h light/12h dark conditions. Phytotoxicity data taken 22 days after treatment for species #1-8 and 12 days for species #9-12 are presented in Tables 5 and 6, respectively. The rating scale for both tables is shown in Table 4:

**Table 4. Rating Scale**

| **Rating Scale** | **% Control** |
|---|---|
| 0 | 0 |
| 1 | <10 |
| 2 | 25 |
| 3 | 50 |
| 4 | 75 |
| 5 | 100 |

**Table 5. Phytotoxicity Data for Species #1-8**

| **Treatment** | **Horseweed** | **Lambsguarter** | **Dandelion** | **Curlydock** | **Crabgrass** | **Mustard** | **Nightshade** | **Bluegrass** |
|---|---|---|---|---|---|---|---|---|
| UTC | 0.0 | 0.0 | 0.0 | 0.0 | 0.0 | 0.7 | 0.0 | 0.0 |
| 1.25mg/mL | 0.0 | 4.7 | 0.0 | 0.0 | 0.0 | 4.3 | 0.0 | 0.0 |
| 2.5 mg/mL | 0.7 | 4.5 | 0.0 | 0.0 | 0.0 | 4.7 | 0.0 | 0.0 |
| 5.0mg/mL | 4.3 | 5.0 | 0.0 | 0.0 | 0.0 | 5.0 | 0.0 | 0.0 |
| 10.0 mg/mL | 4.7 | 5.0 | 0.0 | 0.0* | 0.0 | 5.0 | 1.5 | 0.0 |
| Roundup | 5.0 | 5.0 | 5.0 | 5.0 | 5.0 | 5.0 | 5.0 | 5.0 |
| | * stunting that resulted in plants approximately half the size of untreated plants | | | | | | | |

**Table 6. Phytotoxicity Data for Species #9-12**

| **Treatment** | **Cocklebur** | **Foxtail** | **Bermuda Grass** | **Sowthistle** | **Mallow** |
|---|---|---|---|---|---|
| UTC | 0.0 | 0.7 | 0.0 | 0.0 | 2.8 |
| 1.25mg/mL | 0.5 | 0.3 | 0.3 | 0.0 | 2.0 |
| 2.5mg/mL | 0.5 | 0.7 | 0.5 | 0.0 | 2.7 |
| 5.0 mg/mL | 0.8 | 0.3 | 0.2 | 0.0 | 2.2 |
| 10.0 mg/mL | 0.7 | 0.7 | 0.3 | 0.2 | 1.7 |
| Roundup | 4.7 | 4.8 | 4.7 | 5.0 | 5.0 |

Based on the results obtained in these studies, the compounds extracted from fermentation broths of the isolated *Burkholderia* species had herbicidal activity against several weed species are tested. Of the twelve species tested, Lambsquarters and mustard are most susceptible, followed by mallow and horseweed. Extract concentration as low as 1.25 mg/mL is able to provide almost complete control of Lambsquarters and mustard, whereas higher concentration is required for the mallow and horseweed.

In a separate experiment, using the same design as described above, systemic activity is tested. A 10 mg/ml crude extract supernatant of *Burkholderia sp. A396* is painted onto first true leaves of Ragweed, Mustard, Nightshade, Crabgrass, Wheat and Barnyard Grass. Seedlings are evaluated 7 days after treatment. Observed symptoms include: burning, warping, bleaching Herbicidal activity is observed in the next leaf above the treated leaf in Ragweed, Mustard and Nightshade. No systemic activity is observed in the tested grasses. In a second experiment. five fractions of the same crude extract (10 mg/ml) are evaluated using the same experimental design as described above. Seedlings of Mustard, Wheat and Crabgrass are treated. Seven and 20 days after treatment, symptoms of herbicidal activity are observed in Mustard from four out of the five fractions (091113B4F6, 091113B4F7,091113B4F8 and 091113B4F9) using a C-18 column (Phenomenex Sepra C18-E, 50 *µ*m, 65Å). Symptoms are observed in the next leaf above the treated leaf. No systemic activity is observed in the tested grasses.

### 3. Example 3. Burkholderia sp. as an Insecticide

### 3.1. Contact Activity Studies

The following assay is used in the initial screening phase to determine if the compounds derived from a culture of the novel *Burkholderia* species has contact activity against a Lepidopteran pest (larvae). It is further used as a tool for the bioassay-guided fractionation to determine the active fractions and peaks derived from the whole-cell-broth extract. The test is conducted in individual 1.25 oz plastic cups using either Cabbage looper (*Tricoplusia ni*) late third instar larvae or Beet Armyworm (*Spodoptera exigua*) early third instar larvae. A 1cm x 1cm piece of solid Beet armyworm diet is placed in the center of each cup together with one larvae. A 1µl aliquot of each treatment (whole cell broth or extract from a 5-day-old *Burkholderia* A396 culture) is injected on each larvae thorax (dorsal side) using a Hamilton Precision Syringe. Each treatment is replicated ten times. Water is used as a negative control treatment and malathion as the positive control treatment. After injection, each cup is covered with parafilm with an airhole, and the cups are incubated for three days at 26°C. Mortality evaluations are done daily, starting 24 hours after the treatment.

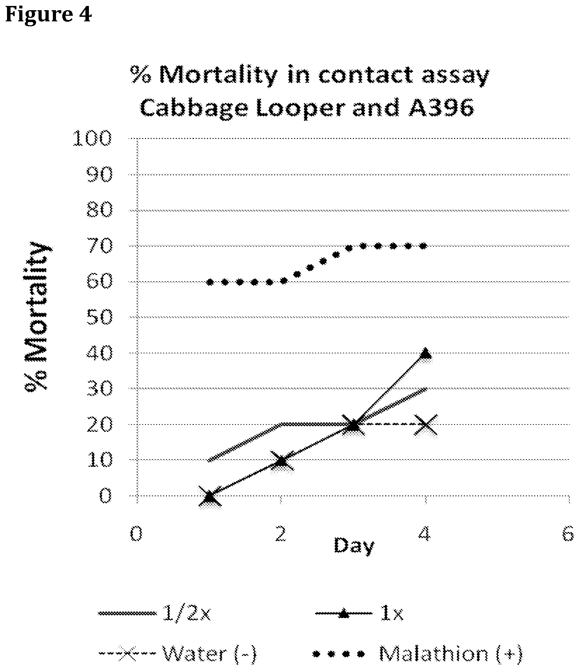

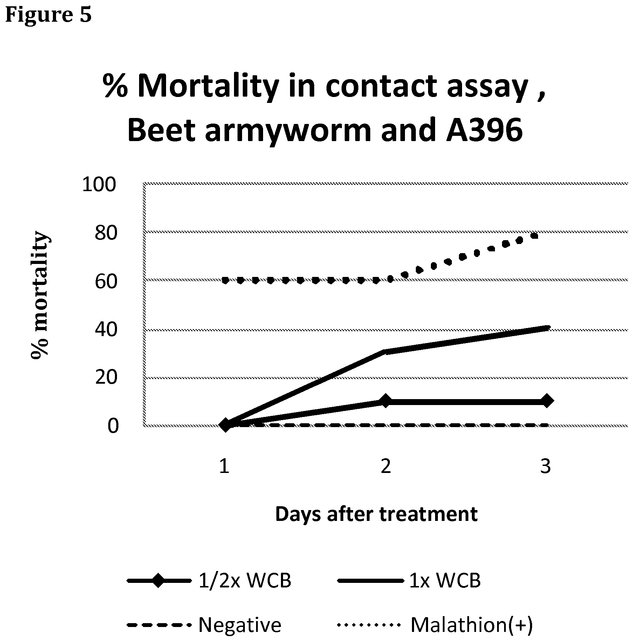

Figures 4 and 5 present the results from contact activity tests. According to the results, the filter-sterilized broth from a *Burkholderia* sp culture killed about 40% of all test insects within 3 days. Diluted broth (50%) has lower activity, resulting in about 10% control in both insects tested.

### 3.2. Activity Against Larvae Through Feeding

Direct toxicity via feeding is tested using the diet-overlay tests with following 96-well plate assay format using microtiter plates with 200 µl of solid, artificial Beet Armyworm diet in each well. One hundred (100) microliters of each test sample is pipetted on the top of the diet (one sample in each well), and the sample is let dry under flowing air until the surface is dry. Each sample (filter-sterilized through a 0.2 micron filter) is tested in six replicates, and water and a commercial Bt (*B. thuringiensis*) product are used as negative and positive controls, respectively. One third instar larvae of the test insect (Cabbage looper-*Trichoplusia ni;* Beet armyworm - *Spodoptera exiqua*; Diamondback Moth - *Plutella xylostella*) is placed in each well, and the plate is covered with plastic cover with airholes. The plates with insects are incubated at 26°C for 6 days with daily mortality evaluations.

Figure 5 represents data from a diet overlay study with Beet Armyworm (*Spodoptera exigua*) early third instar larvae tested at four different broth concentrations:1x (100%) ,1/4x (25%), 1/8x (12.5%), 1/16x (6.125%). The data shows that the undiluted, filter-sterilized broth is able to give 100% control at the end of the 7-day incubation period. Similar control is obtained with a 4-fold dilution of the broth, and in the end of the study, both undiluted and 4-fold diluted broths are comparable to Bt used as a positive control. However, the effect of Bt is significantly faster than that of the *Burkholderia* broths. Efficacy against armyworm larvae is dependent on broth concentration, and the two lowest broth concentrations (12.5% and 6.125%) provided less control than the two highest ones. However, the performance of the 12.5% dilution is not much lower than the 25% dilution. The 16-fold dilution of broth is clearly not efficient enough, and it only provided partial (33%) control of armyworm larvae during this 7-day study. The corresponding mortality rates for the same broth dilution used on cabbage loopers and diamondback moth larvae are a little higher with 6.125% broth killing 80% and 50% of larvae, respectively.

### 3.3. In vitro activity against sucking insects

Five stinkbug *(Euschistus* sp.) adults are placed in each 16 oz plastic container lined with a piece of paper towel. A microcentrifuge tube containing 2 mL of each test sample (filter sterilized whole broth) is capped with a cotton ball, and laid down on the bottom of the plastic container. One sunflower seed is placed next to the tube as bait. Water and a commercial product with a mixture of pyrethrin and PBO at a recommended rate are used as negative and positive controls, respectively. Each container is closed with a lid, and they are incubated at 25°C for 7 days with daily mortality checks.

Results are presented below in Table 7 and they show about 80% control of sucking insect (stinkbug) by day 7 in this *in vitro* system with 50% diluted broth. In this study, the diluted fermentation broth of *Burkholderia* A396 is more effective in controlling stinkbugs than the commercial product used as a positive control. Interestingly, the non-diluted broth resulted in lower insect control, which might be an indication of antifeedant (feeding inhibition) properties of the active secondary metabolites produced by this new species of *Burkholderia.*

**Table 7. Effect of A396 on Stinkbugs**

| **Treatment** | **% control (Day 3)** | **% control (Day 5)** | **% control (Day 7)** |
|---|---|---|---|
| A396 undil. broth (1x) | 0 | 0 | 40 |
| A396 broth dil. 50% (0.5x) | 20 | 20 | 80 |
| Pyrethrin+PBO (pos control) | 0 | 0 | 40 |
| Water (neg control) | 0 | 0 | 0 |

### 4. Example 4. Sucking insect test in vivo

The *in vivo* efficacy of the filtered whole cell broth is tested in a plant assay with mustard plants and green peach aphid (*Myzus persicae*) as the test insect. Approximately one-month-old Florida Broadleaf mustard (*Brassica* sp.) plants are sprayed with two different concentrations (1x and 0.5x) of the filter sterilized whole cell broth of *Burkholderia* sp. using a Paasche airbrush. Water and a commercial product of avermectin (Avid) are used as negative and positive controls, respectively. The plants are allowed to dry on the benchtop, after which they are placed in a 6-cup plastic container with a lid with airholes. Ten aphids at various developmental stages are placed on each test plant, and the plants are incubated under growth lamps for 7 days at 25°C. Daily evaluations for the number of aphids on each plant (summarized in table Table 8 below) are made and recorded in a notebook.

**Table 8. In vivo Efficacy of A396 on Green Peach Aphids**

| **Treatment** | **# live aphids Day 0** | **# live aphids Day 2** | **# live aphids Day 4** | **# live aphids Day 7** |
|---|---|---|---|---|
| A396 undiluted broth (1x) | 10 | 36 | 88 | 145 |
| A396 broth diluted (0.5x) | | 47 | 138 | 217 |
| Avermectin (pos control) | | 0 | 0 | 0 |
| Water (neg control) | 10 | 140 | 364 | 393 |

According to the results, both concentrations of the filter-sterilized broth derived from a culture of a novel species of *Burkholderia* are able to control the population growth of a sucking insect, *M. persicae.*

### 5. Example 5. Nematocidal Activity

### 5.1 Study #1

To assess the effect of filter-sterilized *Burkholderia* sp A396 culture broth on the motility (and subsequent recovery) of juvenile (J2) root-knot nematodes (*Meloidogyne incognita* VW6), the following test is conducted on 24-well plastic cell-culture plates:
A 300-ul aliquot of each test solution (either 1x or 0.5x filter-sterilized broth) is added into appropriate wells after which, fifteen nematodes dispensed in 10 µl of DI water are added into each well, plate is closed with a lid, and incubated at 25°C for 24 hours. Water and Avid at 20,000x dilution are used as negative and positive controls, respectively. Effect of each compound on nematode mobility is checked after 24 hours by probing each nematode with a needle, and the proportion of immobile nematodes in each treatment is recorded in a notebook using a % scale. To assess the recovery of mobility in each treatment, a volume of 200 µl is removed from each well, and the remaining solution in each well is diluted by adding 2 mL of DI water. Plates are again incubated for 24 hours as described above, after which the second mobility evaluation is performed.

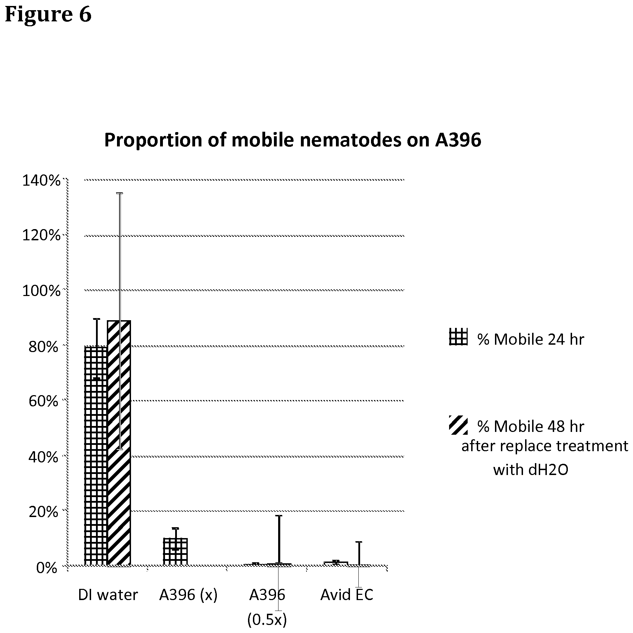

The results presented in Figure 6 show the filter-sterilized broth at both test concentrations can immobilize the free-living juvenile root-knot nematodes. This effect lasts at least for 24-hours, which suggests that Burkholderia A396 broth can be used to prevent plants from nematode infections.

### 5.2 Study #2

### Materials and Methods

Mini Drench Test: *Burkholderia A396* whole cell broth is tested in a greenhouse assay conducted in 45 ml pots. Cucumber seeds cv. Toshka are sown directly into pots filled with a sandy loam soil. Ten days later, pots were each treated with 5 ml of a suspension. Specific amounts used are shown in Table 9:

**Table 9**

| | |
|---|---|
| Compounds | *Burkholderia* strain A396 |
| | Fosthiazate (Standard, EC 150) (positive control) |
| Test species | *Meloidogyne sp.* applied at 3000 eggs per mini drench pot (in 2 ml) |
| Test plant | *Cucumis sativus* (cucumber cv. Toschka) |
| Test formulation | 100% liquid formulation |
| Test concentrations | 100, 50, 25, 12.5, 6, 3, 1.5 ml/L |
| Test application | Drench application |

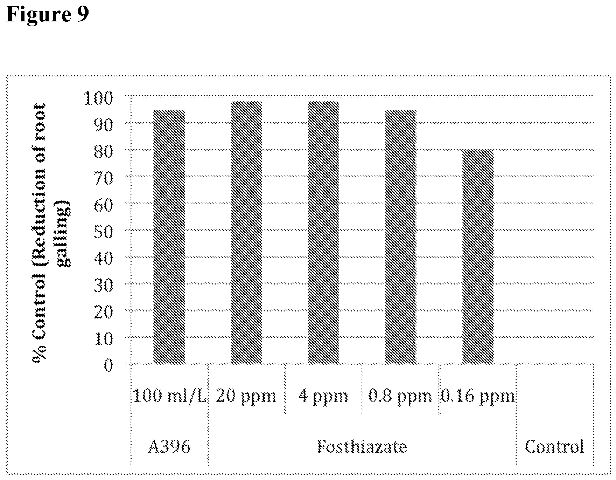

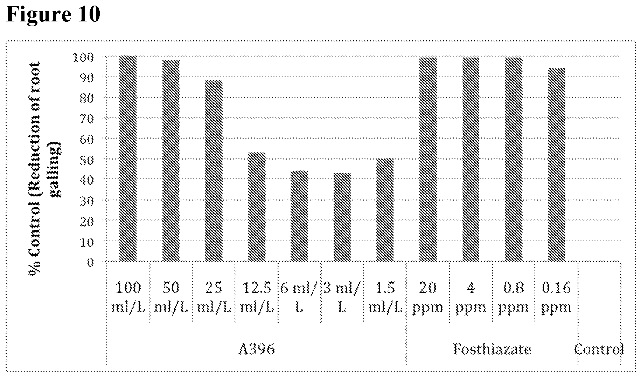

As indicated in Table 9, pots are inoculated with 3000 eggs of *M. incognita.* Four replicates were prepared for each treatment and rate. The trial was harvested fourteen days after trial application and inoculation. Root galling was assessed according to Zeck's gall index (Zeck, 1971). Phytotoxicity was measured as a reduction of root galling in comparison to the control. The results are shown in Figures 9 and 10.

In *Mini Drench Test no.1* (see Figure 9), the activity of the treatment was very high and a reduction of almost 100% was observed when applied at a concentration of 100 ml/L *Burkholderia A396.* Fosthiazate performed as usual (100% control at 20 ppm).

In Mini Drench Test no. 2 (see Figure 10) a 100% reduction of root galling was achieved at the highest concentration of 100 ml/L dropping to approximately 50% at 1.5 ml/L.Fosthiazate performed as usual (100% control at 20 ppm).

### 5.3 Study #3

To demonstrate the nematicidal activity of *Burkholderia A396,* a greenhouse study on cucumber (*Cucumis sativus*) is performed using a whole cell broth of *Burkholderia A396* as the test product to control root knot nematodes (*Meloidogyne incognita*). One cucumber plant per pot is planted in soil and grown in a greenhouse under artificial lights at 28°C. Each pot with a plant is treated with an aliquot (about 80 mL) of either the undiluted test product or a test product diluted to 5% with water. Each *Burkholderia* A396 treatment as well as a positive control treatment with Temik (at a label rate) and a negative control with no additions consisted of five replicates. Plants are grown in a greenhouse for 60 days, after which each plant was harvested and evaluated for fresh shoot and root weights. Number of nematode eggs in each pot was recorded and a parameter indicating the number of eggs per a gram of root mass was calculated. Statistical analysis (ANOVA) is perfomed, and the statistical differences among treatment means at p<0.1 was calculated. Data presented in Table 10 below shows that even though not statistically different from the untreated control, the pots treated with A396 whole cell broth contained less nematode eggs than the untreated control pots. The effect calculated as number of eggs per root mass is more clear when undiluted broth is used as a treatment.

**Table 10. The effect of A396 whole cell broth on the cucumber shoot and root weight, total number of M. incognita eggs per pot and the number of eggs per gram of root mass.**

| | shoot fresh wt | | root fresh wt | | # of eggs | | # of eggs/g of root | |
|---|---|---|---|---|---|---|---|---|
| untreated | 15.22 | b | 11.76 | bc | 67693 | a | 5252.0 | ab |
| A396 5% v/v | 11.89 | b | 6.914 | c | 56084 | a | 8419.4 | a |
| A 396 undiluted | 15.66 | b | 11.09 | bc | 40463 | a | 3929.2 | ab |
| Temik 15 G 5 lb/a | 29.54 | a | 29.74 | a | 68907 | a | 2604.4 | b |
| LSD at p < 0.1 | 5.34 | | 6.9879 | | 36509.2 | | 3317.07 | |

### 6. Example 6. Isolation of Templazole A and B

### Methods and Materials

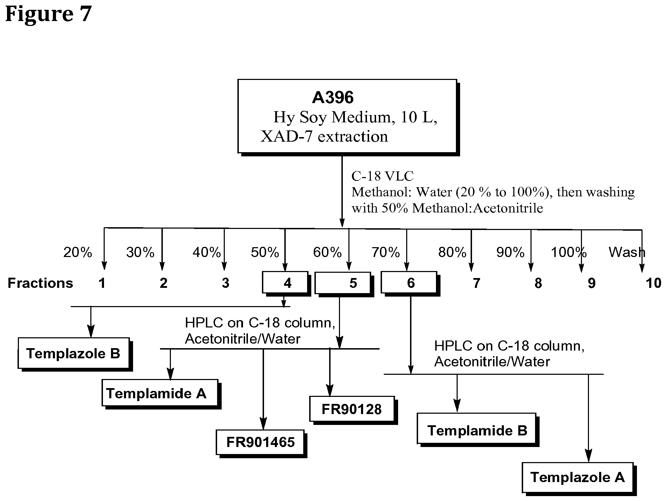

The following procedure is used for the purification of Templazole A and B extracted from cell culture of *Burkholderia sp* (see Figure 7):
The culture broth derived from the 10-L fermentation *Burkholderia* (A396) in Hy soy growth medium is extracted with Amberlite XAD-7 resin (Asolkar et al., 2006) by shaking the cell suspension with resin at 225 rpm for two hours at room temperature. The resin and cell mass are collected by filtration through cheesecloth and washed with DI water to remove salts. The resin, cell mass, and cheesecloth are then soaked for 2 h in acetone after which the acetone is filtered and dried under vacuum using rotary evaporator to give the crude extract. The crude extract is then fractionated by using reversed-phase C18 vacuum liquid chromatography (H₂O/CH₃OH; gradient 90:20 to 0:100%) to give 10 fractions. These fractions are then concentrated to dryness using rotary evaporator and the resulting dry residues are screened for biological activity using 96 well plate lettuce seeding assay. The active fractions are then subjected to reversed phase HPLC (Spectra System P4000 (Thermo Scientific) to give pure compounds, which are then screened in above mentioned bioassays to locate/identify the active compounds. To confirm the identity of the compound, additional spectroscopic data such as LC/MS and NMR is recorded.

The active fraction **4** is purified further by using HPLC C-18 column (Phenomenex, Luna 10u C18(2) 100 A, 250 x 30), water:acetonitrile gradient solvent system (0-10 min; 80% aqueous CH₃CN, 10-25 min; 80 - 65% aqueous CH₃CN, 25-50 min; 65 - 50 % aqueous CH₃CN, 50-60 min; 50-70% CH₃CN, 60-80 min; 70-0% aqueous CH₃CN, 80-85 min; 0 - 20% aqueous CH₃CN) at 8 mL/min flow rate and UV detection of 210 nm, to give templazole B, retention time 46.65 min. The other active fraction **6** is also purified using HPLC C-18 column (Phenomenex, Luna 10u C18(2) 100 A, 250 x 30), water:acetonitrile gradient solvent system (0-10 min; 80 % aqueous CH₃CN, 10-25 min; 80 - 60 % aqueous CH₃CN, 25-50 min; 60 - 40% aqueous CH₃CN, 50-60 min; 40% CH₃CN, 60-80 min; 40-0% aqueous CH₃CN, 80-85 min; 0-20 % aqueous CH₃CN) at 8 mL/min flow rate and UV detection of 210 nm, to give templazole A, retention time 70.82 min.

Mass spectroscopy analysis of pure compounds is performed on a Thermo Finnigan LCQ Deca XP Plus electrospray (ESI) instrument using both positive and negative ionization modes in a full scan mode (m/z 100-1500 Da) on a LCQ DECA XP^{plus} Mass Spectrometer (Thermo Electron Corp., San Jose, CA). Thermo high performance liquid chromatography (HPLC) instrument equipped with Finnigan Surveyor PDA plus detector, autosampler plus, MS pump and a 4.6 mm x 100 mm Luna C18 5 µm column (Phenomenex). The solvent system consists of water (solvent A) and acetonitrile (solvent B). The mobile phase begins at 10% solvent B and is linearly increased to 100% solvent B over 20 min and then kept for 4 min, and finally returned to 10% solvent B over 3 min and kept for 3 min. The flow rate is 0.5 mL/min. The injection volume was 10 µL and the samples are kept at room temperature in an auto sampler. The compounds are analyzed by LC-MS utilizing the LC and reversed phase chromatography. Mass spectroscopy analysis of the present compounds is performed under the following conditions: The flow rate of the nitrogen gas was fixed at 30 and 15 arb for the sheath and aux/sweep gas flow rate, respectively. Electrospray ionization was performed with a spray voltage set at 5000 V and a capillary voltage at 35.0 V. The capillary temperature was set at 400°C. The data was analyzed on Xcalibur software. The active compound templazole A has a molecular mass of 298 and showed *m*/*z* ion at 297.34 in negative ionization mode. The LC-MS chromatogram for templazole B suggests a molecular mass of 258 and exhibited *m*/*z* ion at 257.74 in negative ionization mode.

¹H, ¹³C and 2D NMR spectra were measured on a Bruker 500 MHz & 600 MHz gradient field spectrometer. The reference is set on the internal standard tetramethylsilane (TMS, 0.00 ppm).

For structure elucidation of templazole A, the purified compound with a molecular weight 298 is further analyzed using a 500 MHz NMR instrument, and has ¹H NMR δ values at 8.44, 8.74, 8.19, 7.47, 7.31, 3.98, 2.82, 2.33, 1.08 and has ¹³C NMR δ values of 163.7, 161.2, 154.8, 136.1, 129.4, 125.4, 123.5, 123.3, 121.8, 121.5, 111.8, 104.7, 52.2, 37.3, 28.1, 22.7, 22.7. Templazole A has UV absorption bands at 226, 275, 327 nm, which suggested the presence of indole and oxazole rings. The molecular formula, C₁₇H₁₈N₂O₃, was determined by interpretation of ¹H, ¹³C NMR and HRESI MS data *m*/*z* 299.1396 (M+H)⁺ (Calcd for C₁₇H₁₉N₂O₃, 299.1397), which entails a high degree of unsaturation shown by 10 double bond equivalents. The ¹³C NMR spectrum revealed signals for all 17 carbons, including two methyls, a methoxy, a methylene carbon, an aliphatic methine, an ester carbonyl, and eleven aromatic carbons. The presence of 3'-substituted indole was revealed from ¹H-¹H COSY and HMBC spectral data. The ¹H-¹H COSY and HMBC also indicated the presence of a carboxylic acid methyl ester group and a -CH₂-CH-(CH₃)₂ side chain. From the detailed analysis of ¹H-¹H COSY, ¹³C, and HMBC data it was derived that the compound contained an oxazole nucleus. From the 2D analysis it was found that the isobutyl side chain was attached at C-2 position, a carboxylic acid methyl ester at C-4 position and the indole unit at C-5 position to give templazole A.

The second herbicidally active compound, templazole B, with a molecular weight 258 is further analyzed using a 500 MHz NMR instrument, and has ¹H NMR δ values at 7.08, 7.06, 6.75, 3.75, 2.56, 2.15, 0.93, 0.93 and ¹³C NMR values of δ 158.2, 156.3, 155.5, 132.6, 129.5, 129.5, 127.3, 121.8, 115.2, 115.2, 41.2, 35.3, 26.7, 21.5, 21.5. The molecular formula, is assigned as C₁₅H₁₈N₂O₂, which is determined by interpretation of ¹H, ¹³C NMR and mass data. The ¹³C NMR spectrum revealed signals for all 15 carbons, including two methyls, two methylene carbons, one aliphatic methine, one amide carbonyl, and nine aromatic carbons. The general nature of the structure was deduced from ¹H and ¹³C NMR spectra that showed a *para*-substituted aromatic ring [δ 7.08 (2H, d, *J* = 8.8 Hz), 6.75 (2H, d, *J* = 8.8 Hz), and 132.7, 129.5, 115.2, 127.3, 115.2, 129.5]. The ¹H NMR spectrum of this structure together with the ¹H-¹H COSY and HSQC spectra, displayed characteristic signals for an isobutyl moiety [δ 0.93 (6H, d, *J* = 6.9 Hz), 2.15 (1H, sept., *J* = 6.9 Hz), 2.57 (2H, d, *J* = 6.9 Hz). In addition, an olefinic/aromatic proton at (δ 7.06, s), and a carbonyl carbon group (δ 158.9) were also found in the ¹H and ¹³C NMR spectra. On inspection of the HMBC spectrum, the H-1' signal in the isobutyl moiety correlated with the olefinic carbon (C-2, δ 156.3), and the olefinic proton H-4 correlated with (C-5, δ 155.5; C-2, 156.3 & C-1", 41.2). The methylene signal at δ 3.75 correlated with C-5, C-4 as well as the C-2" of the para-substituted aromatic moiety. All these observed correlations suggested the connectivity among the isobutyl, and the para-substituted benzyl moieties for the skeleton of the structure as shown. In addition, the carboxamide group is assigned at the para position of the benzyl moiety based on the HMBC correlation from the aromatic proton at H-4"& H-6" position. Thus, based on the above data, the structure was designated as templazole B.

### 7. Example 7. Isolation of FR90128

The whole cell broth from the fermentation of *Burkholderia sp.* in an undefined growth medium is extracted with Amberlite XAD-7 resin (Asolkar et al., 2006) by shaking the cell suspension with resin at 225 rpm for two hours at room temperature. The resin and cell mass are collected by filtration through cheesecloth and washed with DI water to remove salts. The resin, cell mass, and cheesecloth are then soaked for 2 h in acetone after which the acetone is filtered and dried under vacuum using rotary evaporator to give the crude extract. The crude extract is then fractionated by using reversed-phase C18 vacuum liquid chromatography (H₂O/CH₃OH; gradient 90:20 to 0:100%) to give 10 fractions. These fractions are then concentrated to dryness using rotary evaporator and the resulting dry residues are screened for biological activity using both insect bioassay as well as herbicidal bioassay. The active fractions are then subjected to reversed/normal phase HPLC (Spectra System P4000; Thermo Scientific) to give pure compounds, which are then screened in herbicidal, insecticidal and nematicidal bioassays described below to locate/identify the active compounds. To confirm the identity of the compound, additional spectroscopic data such as LC/MS and NMR is recorded.

Mass spectroscopy analysis of active peaks is performed on a Thermo Finnigan LCQ Deca XP Plus electrospray (ESI) instrument using both positive and negative ionization modes in a full scan mode (m/z 100-1500 Da) on a LCQ DECA XP^{plus} Mass Spectrometer (Thermo Electron Corp., San Jose, CA). Thermo high performance liquid chromatography (HPLC) instrument equipped with Finnigan Surveyor PDA plus detector, autosampler plus, MS pump and a 4.6 mm x 100 mm Luna C18 5 µm column (Phenomenex). The solvent system consists of water (solvent A) and acetonitrile (solvent B). The mobile phase begins at 10% solvent B and is linearly increased to 100% solvent B over 20 min and then kept for 4 min, and finally returned to 10% solvent B over 3 min and kept for 3 min. The flow rate is 0.5 mL/min. The injection volume is 10 *µ*L and the samples are kept at room temperature in an auto sampler. The compounds are analyzed by LC-MS utilizing the LC and reversed phase chromatography. Mass spectroscopy analysis of the present compounds is performed under the following conditions: The flow rate of the nitrogen gas is fixed at 30 and 15 arb for the sheath and aux/sweep gas flow rate, respectively. Electrospray ionization is performed with a spray voltage set at 5000 V and a capillary voltage at 35.0 V. The capillary temperature is set at 400°C. The data is analyzed on Xcalibur software. Based on the LC-MS analysis, the active insecticidal compound from fraction **5** has a molecular mass of 540 in negative ionization mode.

For structure elucidation, the purified insecticidal compound from fraction **5** with molecular weight 540 is further analyzed using a 500 MHz NMR instrument, and has ¹H NMR values at 6.22, 5.81, 5.69, 5.66, 5.65, 4.64, 4.31, 3.93, 3.22, 3.21, 3.15, 3.10, 2.69, 2.62, 2.26, 2.23. 1.74, 1.15, 1.12, 1.05, 1.02; and has ¹³C NMR values of 172.99, 172.93, 169.57, 169.23, 167.59, 130.74, 130.12, 129.93, 128.32, 73.49, 62.95, 59.42, 57.73, 38.39, 38.00, 35.49, 30.90, 30.36, 29.26, 18.59, 18.38, 18.09, 17.93, 12.51. The NMR data indicates that the compound contains amino, ester, carboxylic acid, aliphatic methyl, ethyl, methylene, oxymethylene, methine, oxymethine and sulfur groups. The detailed 1D and 2D NMR analysis confirms the structure for the compound as FR90128 as a known compound.

### 8. Example 8. Herbicidal activity of FR90128

The herbicidal activity of the active compound FR90128 (MW 540) is tested in a laboratory assay using one-week old barnyard grass (*Echinochloa crus-galli*) seedlings in a 96-well plate platform. One grass seedling was placed in each of the wells containing 99 microliters of DI water. One microliter aliquot of the pure compound in ethanol (10 mg/mL) is added into each well, and the plate is sealed with a lid. One microliter of ethanol in 99 microliters of water is used as a negative control. The treatments were done in eight replicates, and the sealed plate is incubated in a greenhouse under artificial lights (12 hr light/dark cycle). After five days, the results are read. The grass seedlings in all eight wells that received the active compound are dead with no green tissue left, whereas the seedlings in the negative control wells were actively growing.

### 9. Example 9. Insecticidal activity of FR90128

The insecticidal activity of the active compound FR90128 (MW 540) is tested in a laboratory assay using a contact bioassay system. The compound is dissolved in 100% ethanol to concentrations of 0.001, 0.005, 0.01, 0.025, 0.05, 0.1, 0.25, and 0.5 µg/µL. Individual early 3^{rd} instar Beet Armyworm, *Spodoptera exigua*, larvae are placed in 1.25 ounce plastic cups with a 1 cm² piece of artificial diet (Bio-Serv). A Hamilton Micropipette is used to apply 1 µL of compound to the thorax of each larvae. Cups are covered with stretched parafilm and a single hole is cut into the parafilm for aeration. Ten larvae per concentration are treated. The assay is incubated at 25°C, 12h light/12h dark. Larvae are scored at 48 and 72 hours after application. Probit analysis is performed to assess LC₅₀ value which is found for compound (MW 540) as 0.213.

### 10. Example 10. Isolation of Templamide A, B, FR901465 and FR90128

### Methods and Materials

The following procedure is used for the purification of compounds extracted from cell culture of *Burkholderia sp* (see Figure 7):
The culture broth derived from the 10-L fermentation *Burkholderia* (A396) in Hy soy growth medium is extracted with Amberlite XAD-7 resin (Asolkar et al., 2006) by shaking the cell suspension with resin at 225 rpm for two hours at room temperature. The resin and cell mass are collected by filtration through cheesecloth and washed with DI water to remove salts. The resin, cell mass, and cheesecloth are then soaked for 2 h in acetone after which the acetone is filtered and dried under vacuum using rotary evaporator to give the crude extract. The crude extract is then fractionated by using reversed-phase C18 vacuum liquid chromatography (H₂O/CH₃OH; gradient 90:20 to 0:100%) to give 10 fractions. These fractions are then concentrated to dryness using rotary evaporator and the resulting dry residues are screened for biological activity using 96 well plate lettuce seeding (herbicidal) and early 3^{rd} instar Beet Armyworm (insecticidal) assay. The active fractions are then subjected to repeatedly to reversed phase HPLC separation (Spectra System P4000 (Thermo Scientific) to give pure compounds, which are then screened in above-mentioned bioassays to locate/identify the active compounds. To confirm the identity of the compound, additional spectroscopic data such as LC/MS, HRMS and NMR are recorded.

The active fraction **5** is purified further by using HPLC C-18 column (Phenomenex, Luna 10u C18(2) 100 A, 250 x 30), water:acetonitrile gradient solvent system (0-10 min; 80 % aqueous CH₃CN, 10-25 min; 80 - 65 % aqueous CH₃CN, 25-50 min; 65 - 50 % aqueous CH₃CN, 50-60 min; 50 - 70 % aqueous CH₃CN, 60-80 min; 70 - 0 % aqueous CH₃CN, 80-85 min; 0 - 20 % aqueous CH₃CN) at 8 mL/min flow rate and UV detection of 210 nm, to give templamide A, retention time 55.64 min and FR901465, retention time 63.59 min and FR90128, retention time 66.65 min respectively. The other active fraction **6** is also purified using HPLC C-18 column (Phenomenex, Luna 10u C18(2) 100 A, 250 x 30), water:acetonitrile gradient solvent system (0-10 min; 70-60 % aqueous CH₃CN, 10-20 min; 60-40 % aqueous CH₃CN, 20-50 min; 40 - 15 % aqueous CH₃CN, 50-75 min; 15 - 0 % CH₃CN, 75-85 min; 0 - 70 % aqueous CH₃CN) at 8 mL/min flow rate and UV detection of 210 nm, to give templamide B, retention time 38.55 min.

Mass spectroscopy analysis of pure compounds is performed on a Thermo Finnigan LCQ Deca XP Plus electrospray (ESI) instrument using both positive and negative ionization modes in a full scan mode (*m*/*z* 100-1500 Da) on a LCQ DECA XP^{plus} Mass Spectrometer (Thermo Electron Corp., San Jose, CA). Thermo high performance liquid chromatography (HPLC) instrument equipped with Finnigan Surveyor PDA plus detector, autosampler plus, MS pump and a 4.6 mm x 100 mm Luna C18 5 µm column (Phenomenex) is used. The solvent system consists of water (solvent A) and acetonitrile (solvent B). The mobile phase begins at 10% solvent B and is linearly increased to 100% solvent B over 20 min and then kept for 4 min, and finally returns to 10% solvent B over 3 min and kept for 3 min. The flow rate is 0.5 mL/min. The injection volume is 10 µL and the samples are kept at room temperature in an auto sampler. The compounds are analyzed by LC-MS utilizing the LC and reversed phase chromatography. Mass spectroscopy analysis of the present compounds is performed under the following conditions: The flow rate of the nitrogen gas is fixed at 30 and 15 arb for the sheath and aux/sweep gas flow rate, respectively. Electrospray ionization is performed with a spray voltage set at 5000 V and a capillary voltage at 45.0 V. The capillary temperature is set at 300°C. The data is analyzed on Xcalibur software. The active compound templamide A has a molecular mass of 555 based on the *m*/*z* peak at 556.41 [M + H]⁺ and 578.34 [M + Na]⁺ in positive ionization mode. The LC-MS analysis in positive mode ionization for templamide B suggests a molecular mass of 537 based *m*/*z* ions at 538.47 [M + H]⁺ and 560.65 [M + Na]⁺. The molecular weight for the compounds FR901465 and FR90128 are assigned as 523 and 540 respectively on the basis of LCMS analysis.

¹H, ¹³C and 2D NMR spectra are measured on a Bruker 600 MHz gradient field spectrometer. The reference is set on the internal standard tetramethylsilane (TMS, 0.00 ppm).

For structure elucidation of templamide A, the purified compound with molecular weight 555 is further analyzed using a 600 MHz NMR instrument, and has ¹H NMR δ values at 6.40, 6.39, 6.00, 5.97, 5.67, 5.54, 4.33, 3.77, 3.73, 3.70, 3.59, 3.47, 3.41, 2.44, 2.35, 2.26, 1.97, 1.81, 1.76, 1.42, 1.37, 1.16, 1.12, 1.04 and has ¹³C NMR values of δ 173.92, 166.06, 145.06, 138.76, 135.71, 129.99, 126.20, 123.35, 99.75, 82.20, 78.22, 76.69, 71.23, 70.79, 70.48, 69.84, 60.98, 48.84, 36.89, 33.09, 30.63, 28.55, 25.88, 20.37, 18.11, 14.90, 12.81, 9.41. The ¹³C NMR spectrum exhibits 28 discrete carbon signals which are attributed to six methyls, four methylene carbons, and thirteen methines including five *sp*², four quaternary carbons. The molecular formula, C₂₈H₄₅NO₁₀, is determined by interpretation of ¹H, ¹³C NMR and HRESI MS data. The detailed analysis of ¹H-¹H COSY, HMBC and HMQC spectral data reveals the following substructures (I - IV) and two isolated methylene & singlet methyl groups. These substructures are connected later using the key HMBC correlations to give the planer structure for the compound, which has been not yet reported in the literature and designated as templamide A. This polyketide molecule contains two tetrahydropyranose rings, and one conjugated amide. 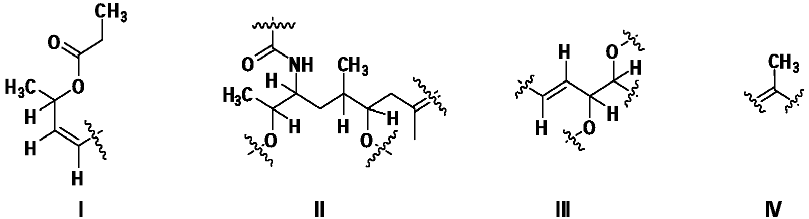 Substructures I-IV assigned by analysis of 1D & 2D NMR spectroscopic data.

The (+) ESIMS analysis for the second herbicidal compound, shows *m*/*z* ions at 538.47 [M + H]⁺ and 560.65 [M + Na]⁺ corresponding to the molecular weight of 537. The molecular formula of C₂₈H₄₃NO₉ is determined by interpretation of the ESIMS and NMR data analysis. The ¹H and ¹³C NMR of this compound is similar to that of templamide A except that a new isolated -CH₂- appear instead of the non-coupled methylene group in templamide A. The small germinal coupling constant of 4.3 Hz is characteristic of the presence of an epoxide methylene group. The presence of this epoxide is further confirmed from the ¹³C NMR shift from 60.98 in templamide A to 41.07 in compound with MW 537. The molecular formulae difference between these two compounds is reasonably explained by elimination of the water molecule followed by formation of epoxide. Thus, on the basis of based NMR and MS analysis the structure for the new compound was assigned and was designated as templamide B.

For structure elucidation, the purified compound from fraction 5 with molecular weight 523 is further analyzed using a 600 MHz NMR instrument, and has ¹H NMR δ values at 6.41, 6.40, 6.01, 5.98, 5.68, 5.56, 4.33, 3.77, 3.75, 3.72, 3.65, 3.59, 3.55, 3.50, 2.44, 2.26, 2.04, 1.96, 1.81, 1.75, 1.37, 1.17, 1.04; and has ¹³C NMR δ values of 172.22, 167.55, 144.98, 138.94, 135.84, 130.14, 125.85, 123.37, 99.54, 82.19, 78.28, 76.69, 71.31, 70.13, 69.68, 48.83, 42.52, 36.89, 33.11, 30.63, 25.99, 21.20, 20.38, 18.14, 14.93, 12.84. The detailed ¹H and ¹³C NMR analysis of compound suggested that this compound was quite similar to compound templamide B; the only difference was in the ester side chain; an acetate moiety was present instead of a propionate moiety in the side chain. The detailed 1D and 2D NMR analysis confirm the structure for the compound as FR901465 as a known compound.

Based on the LC-MS analysis, the other compound from fraction **5** has a molecular mass of 540 in negative ionization mode. For structure elucidation, the purified compound from fraction **5** with molecular weight 540 is further analyzed using a 500 MHz NMR instrument, and has ¹H NMR δ values at 6.22, 5.81, 5.69, 5.66, 5.65, 4.64, 4.31, 3.93, 3.22, 3.21, 3.15, 3.10, 2.69, 2.62, 2.26, 2.23. 1.74, 1.15, 1.12, 1.05, 1.02; and has ¹³C NMR values of 172.99, 172.93, 169.57, 169.23, 167.59, 130.74, 130.12, 129.93, 128.32, 73.49, 62.95, 59.42, 57.73, 38.39, 38.00, 35.49, 30.90, 30.36, 29.26, 18.59, 18.38, 18.09, 17.93, 12.51. The NMR data indicates that the compound contains amino, ester, carboxylic acid, aliphatic methyl, ethyl, methylene, oxymethylene, methine, oxymethine and sulfur groups. The detailed 1D and 2D NMR analysis confirm the structure for the compound as FR90128 as a known compound.

### 11. Example 11. Herbicidal activity of Templamide A, Templamide B, FR901465 and FR90128

The herbicidal activity of templamide A, B, FR901465 and FR90128 are tested in a laboratory assay using one-week old barnyard grass (*Echinochloa crus-galli*) and lettuce (*Lactuca sativa* L.) seedlings in a 96-well plate platform. One seedling is placed in each of the wells containing 99 microliters of DI water. Into each well, a one microliter aliquot of the pure compound in ethanol (10 mg/mL) is added, and the plate is sealed with a lid. One microliter of ethanol in 99 microliters of water is used as a negative control. The treatments are done in eight replicates, and the sealed plate is incubated in a greenhouse under artificial lights (12 hr light/dark cycle). After five days, the results are read. The grass seedlings in all eight wells that received the active compound are dead with no green tissue left, whereas the seedlings in the negative control wells are actively growing. The herbicidal activity of templamide A against lettuce seedlings is slightly lower than for the grass. On the other hand, templamide B provides a better (100%) control of lettuce seedlings (used as a model system for broadleaf weeds) than templamide A (Table 11).

**Table 11: Herbicidal Bioassay data for Templamide A, B, FR901465 and FR90128**

| Compounds¹ | Grass seedlings (% Mortality) | Lettuce seedlings (% Mortality) |
|---|---|---|
| Templamide A | 100 | 88 |
| Templamide B | 0 | 75 |
| FR901465 | 88 | 100 |
| FR90128 | 100 | 88 |
| Control | 0 | 0 |

| | | |
|---|---|---|
| ¹10 µg/mL concentration per well | | |

### 12. Example 12. Insecticidal activity of active compounds

The insecticidal activity of templamide A, B, FR901465 and FR90128 are tested in a laboratory assay using a 96-well diet overlay assay with 1^{st} instar Beet Armyworm larvae using microtiter plates with 200 µl of solid, artificial Beet Armyworm diet in each well. One hundred (100) µl of each test sample is pipetted on the top of the diet (one sample in each well), and the sample is let dry under flowing air until the surface is dry. Each sample was tested in six replicates, and water and a commercial Bt (*B. thuringiensis*) product are used as negative and positive controls, respectively. One first instar larvae of the test insect (Beet armyworm - *Spodoptera exiqua*) was placed in each well, and the plate was covered with plastic cover with airholes. The plates with insects were incubated at 26°C for 6 days with daily mortality evaluations. Based on the results presented in Table 12, templamide A and B results in 40% and 80% mortality, respectively.

**Table 12: Insecticidal Bioassay data for Templamide A, B, FR901465 and FR90128 against 1^{st} instar Beet Army Worm (Spodoptera exigua).**

| Compounds¹ | (% Mortality) |
|---|---|
| Templamide A | 40 |
| Templamide B | 80 |
| FR901465 | 50 |
| FR90128 | 90 |
| Bt | 100 |
| Control | 0 |

| | |
|---|---|
| ¹10 µg/mL concentration per well | |

### Example 11. Fungicidal activity of FR90128 (MW 540)

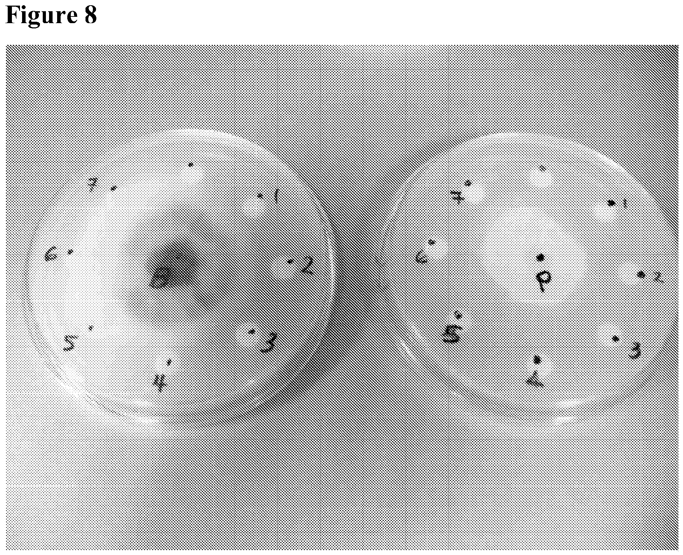

Fungicidal activity of FR90128 (MW 540) against three plant pathogenic fungi (*Botrytis cinerea, Phytophtora* sp., *Monilinia fructicola*) is tested in an in vitro PDA (potato dextrose agar) plate assay. Plates are inoculated with the fungus using a plug method. After the fungus had established and started to grow on the growth medium, eight sterile filter paper disks are placed on each plate about 1 cm from the edge in a circle. Ten microliters of ethanol solution containing 20, 15, 10, 7.5, 5, 2.5 1.25 mg FR90128/mL is added into filter paper disks, and the solution is left to evaporate. One disk imbedded with 10 µL of pure ethanol is used as a negative control. The assay is done with three replicates. Plates are incubated at room temperature for 5 days, after which the fungicidal activity is recorded by measuring the inhibition zone around each filter paper disk corresponding to different concentrations of FR90128. According to the results, FR90128 has no effect on the growth of *Monilinia* but it is effective in controlling the hyphal growth of both *Botrytis* and *Phytophtora.* There seems to be a clear dose-response in inhibition with threshold concentrations of 10 mg/mL and 1.25 mg/mL for *Botrytis* and *Phytophtora*, respectively (Figure 8).

### DEPOSIT OF BIOLOGICAL MATERIAL

The following biological material has been deposited under the terms of the Budapest Treaty with the Agricultural Research Culture Collection (NRRL), 1815 N. University Street, Peoria, Illinois 61604 USA, and given the following number:

| Deposit | Accession Number | Date of Deposit |
|---|---|---|
| *Burkholderia sp.* A396 | NRRL B-50319 | September 15, 2009 |

The strain has been deposited under conditions that assure that access to the culture will be available during the pendency of this patent application to one determined by the Commissioner of Patents and Trademarks to be entitled thereto under 37 C.F.R. §1.14 and 35 U.S.C. §122. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by government action.

The invention described and claimed herein is not to be limited in scope by the specific aspects herein disclosed, since these aspects are intended as illustrations of several aspects of the invention. In the case of conflict, the present disclosure including definitions will control.

### Literature cited:

Anderson, et al. "The structure of thiostrepton," Nature 225: 233-235. 1970.
Andra, "Endotoxin-like properties of a rhamnolipid exotoxin from Burkholderia (Pseudomonas) plantarii: immune cell stimulation and biophysical characterization." Biol. Chem. 387: 301-310. 2006.
Arena, et al. "The mechanism of action of avermectins in Caenorhabditis elegans - correlation between activation of glutamate-sensitive chloride current, membrane binding and biological activity." J Parasitol. 81: 286-294. 1995.
Asolkar, et al., "Weakly cytotoxic polyketides from a marine-derived Actinomycete of the genus Streptomyces strain CNQ-085." J. Nat. Prod. 69:1756-1759. 2006.
Burkhead, et al., "Pyrrolnitrin production by biological control agent Pseudomonas cepacia B37w in culture and in colonized wounds of potatoes." Appl. Environ. Microbiol. 60: 2031-2039. 1994.
Burkholder, W. H "Sour skin, a bacterial rot of onion bulbs." Phytopathology 40: 115-117.1950.
Caballero-Mellado et al., "Burkholderia unamae sp. nov., an N2-fixing rhizospheric and endophytic species." Int. J. Syst. Evol. Microbiol. 54: 1165-1172. 2004.
Cashion et al. "Rapid method for base ratio determination of bacterial DNA." Anal. Biochem. 81: 461-466. 1977.
Casida, et al., US Patent No. 6,689,357.
Chen et al., "Burkholderia nodosa sp. nov., isolated from root nodules of the woody Brazilian legumes Mimosa bimucronata and Mimosa scabrella" Int. J. Syst. Evol. Microbiol. 57: 1055-1059. 2007.
Cheng, A. C. and Currie, B. J. "Melioidosis: epidemiology, pathophysiology, and management." Clin. Microbiol. 18: 383-416. 2005.
Coenye, T. and P. Vandamme, P. "Diversity and significance of Burkholderia species occupying diverse ecological niches." Environ. Microbiol. 5: 719-729. 2003.
Compant, et al. "Diversity and occurence of Burkholderia spp. in the natural environment." FEMS Microbiol. Rev. 32: 607-626. 2008.
De Ley et al. "The quantitative measurement of DNA hybridization from renaturation rates." Eur. J. Biochem. 12: 133-142. 1970.
Duke et al. "Natural products as sources for herbicides: current status and future trends." Weed Res 40: 99-111. 2000.
Gerwick et al., US Patent No. 7,393,812.
Gottlieb et al., US Patent No. 4,808,207.
Gouge et al., US Patent Application Pub. No. 2003/0082147.
Guella et al. "Almazole C, a new indole alkaloid bearing an unusually 2,5-disubstituted oxazole moiety and its putative biogenetic precursors, from a Senegalese Delesseriacean sea weed." Helv. Chim. Acta 77: 1999-2006. 1994.
Guella et al. "Isolation, synthesis and photochemical properties of almazolone, a new indole alkaloid from a red alga of Senegal." Tetrahedron. 62: 1165-1170. 2006.
Henderson, P. J. and Lardy H. A. "Bongkrekic acid. An inhibitor of the adenine nucleotide translocase of mitochondria." J. Biol. Chem. 245: 1319-1326. 1970.
Hirota et al. "Isolation of indolmycin and its derivatives as antagonists of L-tryptophan." Agri. Biol Chem. 42: 147-151. 1978.
Hu, F.-P. and Young, J. M. "Biocidal activity in plant pathogenic Acidovorax, Burkholderia, Herbaspirillum, Ralstonia, and Xanthomonas spp." J. Appl. Microbiol. 84: 263-271.1998.
Huss et al. "Studies of the spectrophotometric determination of DNA hybridization from renaturation rates." System. Appl. Microbiol. 4: 184-192. 1983.
Jansiewicz, W. J. and Roitman J. "Biological control of blue mold and gray mold on apple and pear with Pseudomonas cepacia." Phytopathology 78: 1697-1700. 1988.
Jeddeloh et al., WO2001/055398.
Jansen et al. "Thiangazole: a novel inhibitor of HIV-1 from Polyangium Spec." Liebigs Ann. Chem. 4: 357-3359. 1992.
Jeong et al. "Toxoflavin produced by Burkholderia glumae causing rice grain rot is responsible for inducing bacterial wilt in many field crops." Plant Disease 87: 890-895. 2003.
Knudsen, G. R. and Spurr, J. "Field persistence and efficacy of five bacterial preparations for control of peanut leaf spot." Plant Disease 71: 442-445. 1987.
Koga-Ban et al. "cDNA sequences of three kinds of beta-tubulins from rice." DNA Research 2: 21-26. 1995.
Koide et al. US Patent Application Pub. No. 2008/0096879.
Koyama et al. "Isolation, characterization, and synthesis of pimprinine, pimrinrthine, and pimprinaphine, metabolites of Streptoverticillium olivoreticuli." Agri. Biol. Chem. 45: 1285-1287.1981.
Krieg et al. "Bacillus thuringiensis var. tenebrionis: Ein neuer, gegenuber Larven von Coleopteren wirksamer Pathotyp." Z. Angew. Entomol._96:500-508. 1983.
Kunze et al. "Thiangazole, a new thiazoline antibiotic from Polyangium sp (Myxobacteria Production, antimicrobial activity and mechanism of action." J. Antibiot., 46: 1752-1755. 1993.
Leahy et al. "Comparison of factors influencing trichloroethylene degradation by toluene-oxidizing bacteria." Appl. Environ. Microbiol. 62: 825-833. 1996.
Lessie et al. "Genomic complexity and plasticity of Burkholderia cepacia." FEMS Microbiol. Lett. 144: 117-128. 1996.
Lindquist, N. et al. "Isolation and structure determination of diazonamides A and B, unusual cytotoxic metabolites from the marine ascidian Diazona chinensis." J. Am Chem. Soc. 113: 2303-2304. 1991.
Lorch, H et al. "Basic methods for counting microoganisms in soil and water. In Methods in applied soil microbiology and biochemistry. K. Alef and P. Nannipieri. Eds. San Diego, CA, Academic Press: pp. 146-161. 1995.
Ludovic et al. "Burkholderia diveristy and versatility: An inventory of the extracellular products." J. Microbiol. Biotechnol. 17: 1407-1429. 2007.
Lydon, J. and Duke, S. "Inhibitors of glutamine biosynthesis." in Plant amino acids: Biochemistry and Biotechnology. B. Singh., Ed. New York, USA, Marcel Decker. pp. 445-464. 1999.
Mahenthiralingam et al. "DNA-based diagnostic approaches for identification of Burkholderia cepacia complex, Burkholderia vietnamiensis, Burkholderia multivorans, Burkholderia stabilis, and Burkholderia cepacia genomovars I and III." J.Clin. Microbiol. 38: 3165-3173. 2000.
Ming, L.-J. and Epperson. "Metal binding and structure-activity relationship of the metalloantibiotic peptide bacitracin." Biochemistry 91: 46-58. 2002.
Morita et al. "Biological activity of tropolone." Biol. Pharm. Bull. 26: 1487-1490. 2003.
Nagamatsu, T. "Syntheses, transformation, and biological activities of 7-azapteridine antibiotics: toxoflavin, fervenulin, reumycin, and their analogs". Recent Res. Devel. Org. Bioorg. Chem. 4: 97 -121. 2001.
Naik et al., "Pimprine, an extracellular alkaloid produced by Streptomyces CDRIL-312: fermentation, isolation and pharmacological activity." J. Biotech. 88: 1-10. 2001.
Nakajima et al., "Antitumor Substances, FR901463, FR901464 and FR901465. I. Taxonomy, Fermentation, Isolation, Physico-chemical Properties and Biological Activities." J. Antibiot. 49: 1196-1203.1996.
Nakajima et al. US Patent No. 5,545,542.
Nakajima et al., "Hydantocidin: a new compound with herbicidal activity." J Antibiot. 44: 293-300. 1991.
N'Diaye, I. et al., "Almazole A and amazole B, unusual marine alkaloids of an unidentified red seaweed of the family Delesseriaceae from the coasts of Senegal." Tet Lett. 35: 4827-4830. 1994.
N'Diaye, I. et al., "Almazole D, a new type of antibacterial 2,5-disubstituted oxazolic dipeptide from a red alga of the coast of Senegal." Tet Lett. 37: 3049-3050. 1996.
Nierman et al., "Structural flexibility in the Burkholderia mallei genome." Proc. Natl. Acad. Sci. USA 101: 14246-14251. 2004.
Okazaki et al., "Rhizobial strategies to enhance symbiotic interaction: Rhizobitoxine and 1-aminocyclopropane-1-carboxylate deaminase." Microbes Environ. 19: 99-111. 2004.
Parke, J. L. and D. Gurian-Sherman, D. 2001. "Diversity of the Burkholderia cepacia complex and implications for risk assessment of biological control strains." Annual Reviews in Phytopathology 39: 225-258. 2001.
Parke, et al. US Patent No. 6,077,505.
Pettit, G. et al. "Isolation of Labradorins 1 and 2 from Pseudomonas syringae." J. Nat. Prod. 65: 1793-1797. 2002.
Pitt, et al., "Type characterization and antibiotic susceptibility of Burkholderia (Pseudomonas) cepacia isolates from patients with cystic fibrosis in the United Kingdom and the Republic of Ireland." J. Med. Microbiol. 44: 203-210. 1996.
Ramette et al., "Species abundance and diversity of Burkholderia cepacia complex in the environment." Appl. Environ. Microbiol. 71: 1193-1201. 2005.
Resi et al., "Burkholderia tropica sp. nov., a novel nitrogen-fixing, plant-associated bacterium."Int. J. Syst. Evol. Microbiol. 54: 2155-2162. 2004.
Salama et al. "Potency of spore-gamma-endotoxin complexes of Bacillus thuringiensis against some cotton pests." Z. Angew. Entomol. 91: 388-398. 1981.
Selva et al., "Targeted screening for elongation factor Tu binding antibiotics." J. Antibiot. 50: 22-26. 1997.
Takahashi, S. et al. "Martefragin A, a novel indole alkaloid isolated from a red alga, inhibits lipid peroxidation." Chem Pharm. Bull. 46: 1527-1529. 1998.
Thompson et al. "Spinosad - a case study: an example from a natural products discovery programme." Pest Management Science 56: 696-702. 2000.
Takita et al., "Chemistry of Bleomycin. XIX Revised structures of bleomycin and phleomycin." J. Antibiot. 31: 801-804. 1978.
Tran Van et al., "Repeated beneficial effects of rice inoculation with a strain of Burkholderia vietnamiensis on early and late yield component in low fertility sulphate acid soils of Vietnam." Plant and Soil 218: 273-284. 2000.
Tsuruo et al., "Rhizoxin, a macrocyclic lactone antibiotic, as a new antitumor agent against human and murine tumor cells and their vincristine-resistant sublines." Cancer Res. 46: 381-385. 1986.
Ueda et al., US Patent No. 7,396,665.
Umehara, K. et al. "Studies of new antiplatelet agents WS-30581 A and B." J. Antibiot. 37: 1153-1160. 1984.
Vandamme et al. Polyphasic taxonomic study of the emended genus Arcobacter with Arcobacter butzleri comb. nov. and Arcobacter skirrowii sp. nov., an aerotolerant bacterium isolated from veterinary specimens." Int. J. Syst. Bacteriol. 42: 344-356. 1992.
Vanderwall et al., "A model of the structure of HOO-Co•bleomycin bound to d(CCAGTACTGG): recognition at the d(GpT)site and implications for double-stranded DNA cleavage, Chem. Biol. 4: 373-387. 1997.
Vermis K., et al. "Evaluation of species-specific recA-based PCR tests for genomovar level identification within the Burkholderia cepacia complex." J. Med. Microbiol 51: 937-940. 2002.
Watanabe, H. et al. "A new antibiotic SF2583A, 4-chloro-5-(3'indoly)oxazole, produced by Streptomyces." Meiji Seika Kenkyu Nenpo 27: 55-62. 1988.
Wayne et al., "Report of the Ad Hoc committee on reconciliation of approaches to bacterial systematics." Int. J. Syst. Evol. Microbiol. 37: 463-464. 1987.
Werner et al., "Uptake of indolmycin in gram-positive bacteria." Antimicrob Agents Chemotherapy 18: 858-862. 1980.
Wilson et al. "Toxicity of rhizonin A, isolated from Rhizopus microsporus, in laboratory animals." Food Chem. Toxicol. 22: 275-281. 1984.
Zeck W.M. "Ein Bonitierungsschema zur Feldauswertung von Wurzelgallenbefall. Pflanzenschutznachrichten." Bayer 24,1: 144-147. 1971.
Zhang et al., US Patent No. 7,141,407.
Zhou et al., "Antimicrobial susceptibility and synergy studies of Burkholderia cepacia complex isolated from patients with cystic fibrosis." Antimicrobial Agents and Chemotherapy 51: 1085-088.2007.

## Claims

1. An isolated strain of *Burkholderia* A396 (NRRL Accession No. B-50319) which has the following characteristics:
(A) a 16S rRNA gene sequence comprising the forward sequences having at least 99% identity to the sequences set forth in SEQ ID NO:8, 11, and 12 and reverse sequences having at least 99% identity to the sequences set forth in SEQ ID NO:9, 10, 13, 14 and 15;
(B) pesticidal activity;
(C) produces a pesticidal compound selected from
(i) a compound having a structure (FR901228) 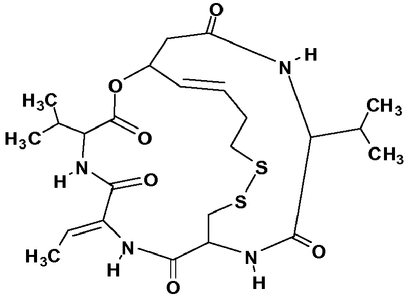
(ii) a compound having a structure 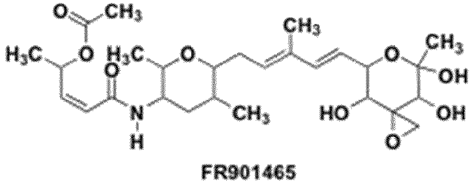
(iii) a compound having a structure 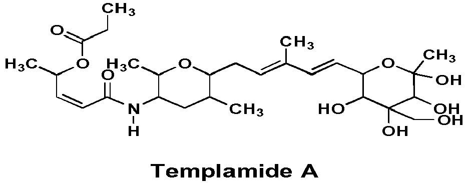
(iv) a compound having a structure 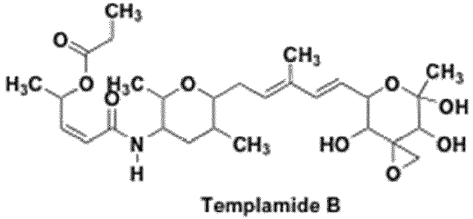
(v) a compound having a structure 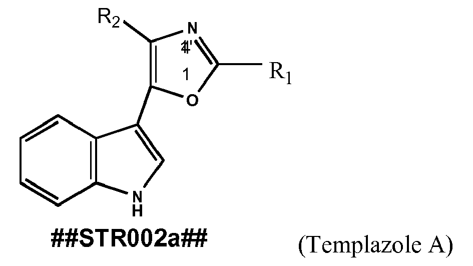 wherein R1 is isobutyl and R2 is carboxylic acid methyl ester; and
(vi) a compound having a structure 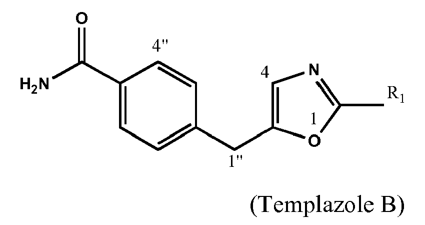 wherein R1 is isobutyl;
(D) is non-pathogenic to vertebrate animals; and
(E) is susceptible to kanamycin, chloramphenicol, ciprofloxacin, piperacillin, imipenem, and a combination of sulphamethoxazole and trimethoprim.

2. An isolated compound having pesticidal activity obtainable from a *Burkholderia* species selected from
(i) a compound having a structure 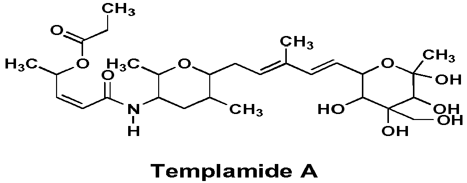
(ii) a compound having a structure 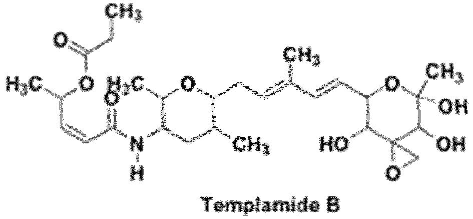
(iii) a compound having a structure 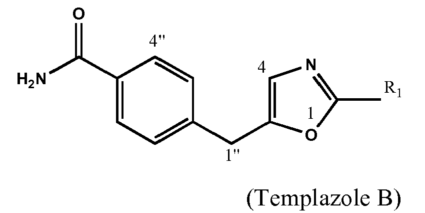 wherein R1 is isobutyl.

3. A method for producing a compound of claim 2, which method comprises culturing the strain of claim 1 and producing said compound.

4. A composition comprising the isolated strain of claim 1 or an isolated compound of claim 2, wherein the composition has pesticidal activity.

5. A method for modulating pest infestation in a plant comprising applying to the plant and/or seeds thereof and/or substrate used for growing said plant an amount of a composition comprising the isolated strain of claim 1, or a composition comprising the isolated compound having pesticidal activity selected from
(i) a compound having a structure 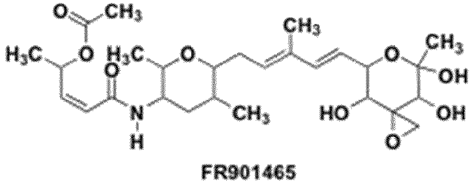
(ii) a compound having a structure 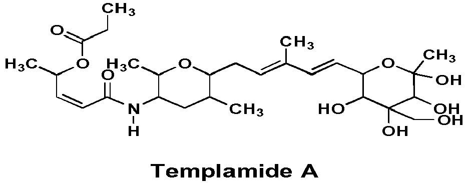
(iii) a compound having a structure 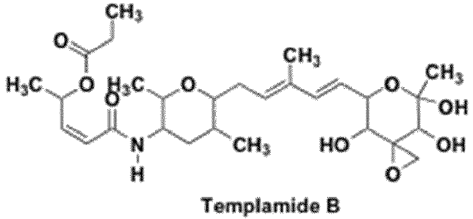
(iv) a compound having a structure 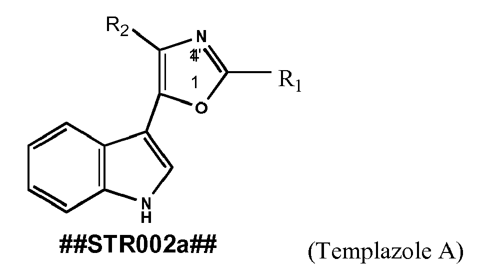 wherein R1 is isobutyl and R2 is carboxylic acid methyl ester; and
(v) a compound having a structure 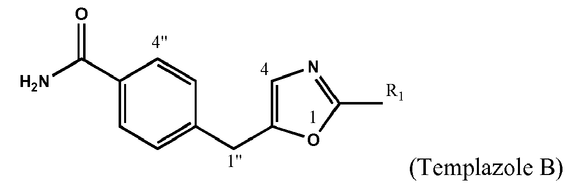
wherein R1 is isobutyl,
effective to modulate said pest infestation.

6. The method according to claim 5, wherein the pest is a fungus.

7. The method according to claim 5, wherein the pest is an insect.

8. The method according to claim 5, wherein the pest is a monocotyledonous, sedge, or dicotyledonous weed.

9. The method according to claim 8, wherein the method modulates emergence and/or growth of the monocotyledonous, sedge, or dicotyledonous weed.

10. The method according to any one of claims 5-9, wherein the composition comprising the isolated strain of claim 1 is applied.

11. The method according to any one of claims 5-9, wherein the composition comprising the isolated compound having the structure 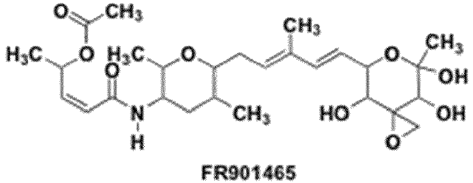 is applied.

12. The method according to any one of claims 5-9, wherein the composition comprising the isolated compound having the structure 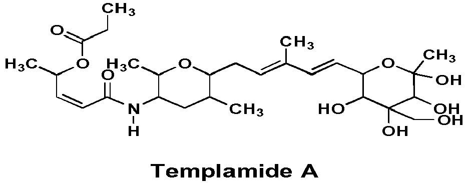 is applied.

13. The method according to any one of claims 5-9, wherein the composition comprising the isolated compound having the structure 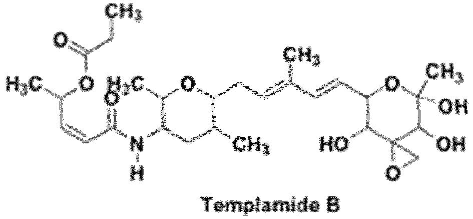 is applied.

14. The method according to any one of claims 5-9, wherein the composition comprising the isolated compound having the structure 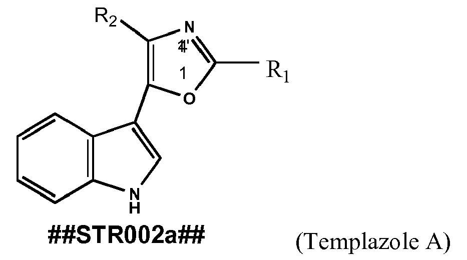 is applied, wherein R1 is isobutyl and R2 is carboxylic acid methyl ester,

15. The method according to any one of claims 5-9, wherein the composition comprising the isolated compound having the structure 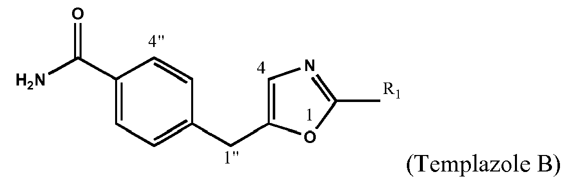 is applied, wherein R1 is isobutyl.

16. A seed comprising the composition comprising the isolated strain of claim 1 or an isolated compound having pesticidal activity selected from
(i) a compound having a structure (FR901228) 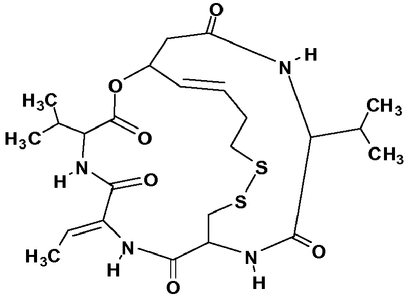
(ii) a compound having a structure 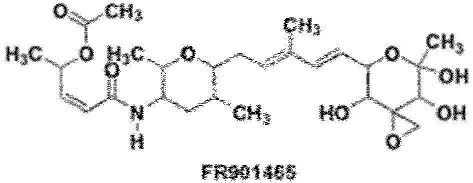
(iii) a compound having a structure 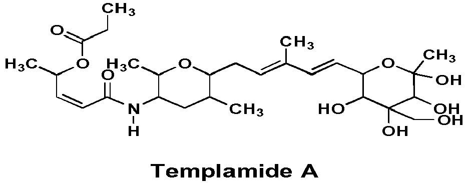
(iv) a compound having a structure 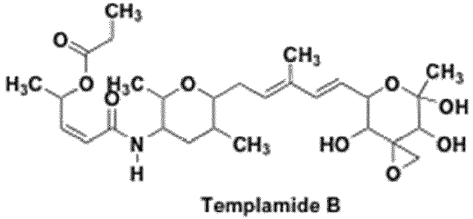
(v) a compound having a structure 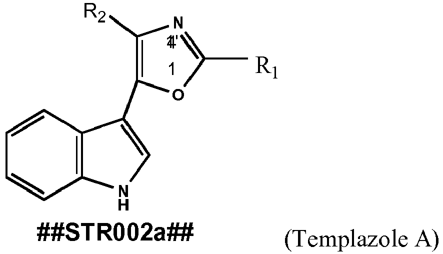 wherein R1 is isobutyl and R2 is carboxylic acid methyl ester; and
(vi) a compound having a structure 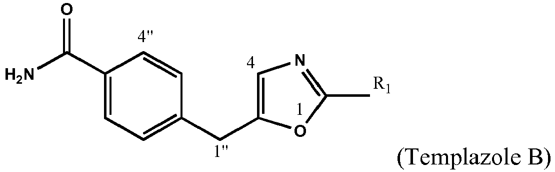 wherein R1 is isobutyl.

## Patentansprüche

1. Isolierter Stamm von *Burkholderia* A396 (NRRL-Zugriffsnummer B-50319), der die folgenden Merkmale aufweist:
(A) eine 16S-rRNA-Gensequenz umfassend die Vorwärtssequenzen mit mindestens 99%iger Identität zu den in SEQ ID Nr. 8, 11 und 12 angegebenen Sequenzen und Umkehrsequenzen mit mindestens 99%iger Identität zu den in SEQ ID Nr. 9, 10, 13, 14 und 15 angegebenen Sequenzen;
(B) pestizide Aktivität;
(C) erzeugt eine pestizide Verbindung ausgewählt aus
(i) einer Verbindung mit einer Struktur (FR901228) 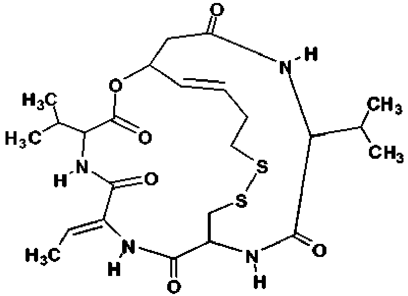
(ii) einer Verbindung mit einer Struktur 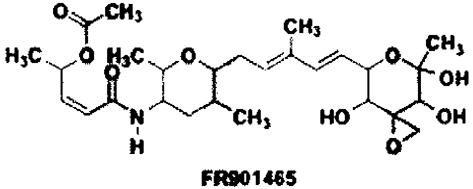
(iii) einer Verbindung mit einer Struktur 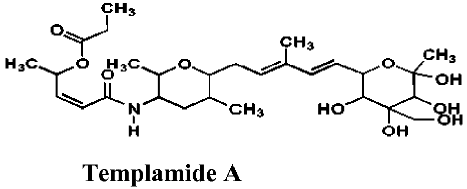
(iv) einer Verbindung mit einer Struktur 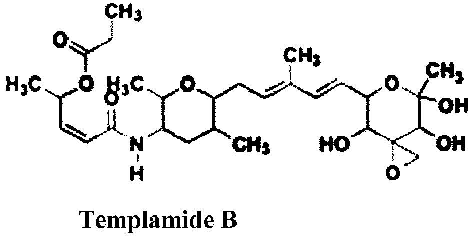
(v) einer Verbindung mit einer Struktur 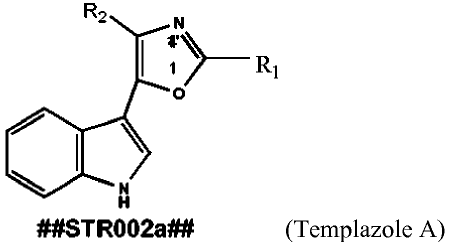 wobei R1 Isobutyl ist und R2 Carboxylsäuremethylester ist; und
(vi) einer Verbindung mit einer Struktur 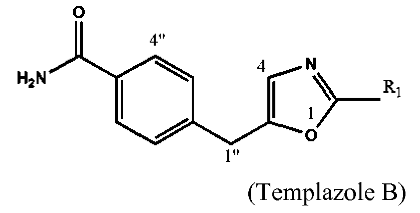 wobei R1 Isobutyl ist;
(D) ist nicht pathogen für Wirbeltiere; und
(E) ist empfindlich gegenüber Kanamycin, Chloramphenicol, Ciprofloxacin, Piperacillin, Imipenem und einer Kombination von Sulphamethoxazol und Trimethoprim.

2. Isolierte Verbindung mit pestizider Aktivität, erhältlich aus einer *Burkholderia*-Spezies, ausgewählt aus
(i) einer Verbindung mit einer Struktur 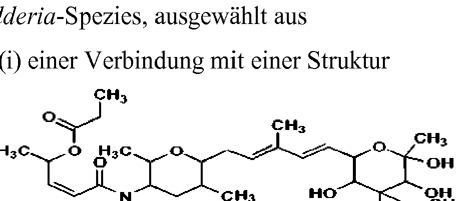
(ii) einer Verbindung mit einer Struktur 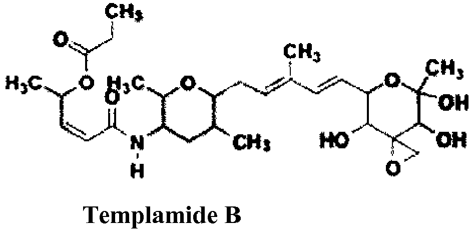
(iii) einer Verbindung mit einer Struktur 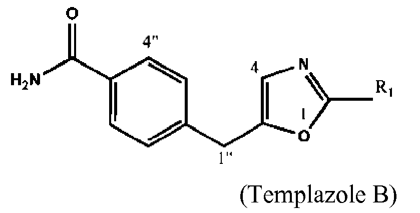 wobei R1 Isobutyl ist.

3. Verfahren zum Herstellen einer Verbindung nach Anspruch 2, wobei das Verfahren das Kultivieren des Stamms nach Anspruch 1 und das Herstellen der Verbindung umfasst.

4. Zusammensetzung umfassend den isolierten Stamm nach Anspruch 1 oder eine isolierte Verbindung nach Anspruch 2, wobei die Zusammensetzung pestizide Aktivität aufweist.

5. Verfahren zum Modulieren des Schädlingsbefalls einer Pflanze, umfassend das Anwenden, auf die Pflanze und/oder Samen davon und/oder zum Anbau der Pflanze verwendetes Substrat, einer Menge einer Zusammensetzung umfassend den isolierten Stamm nach Anspruch 1 oder einer Zusammensetzung umfassend die isolierte Verbindung mit pestizider Aktivität, ausgewählt aus
(i) einer Verbindung mit einer Struktur 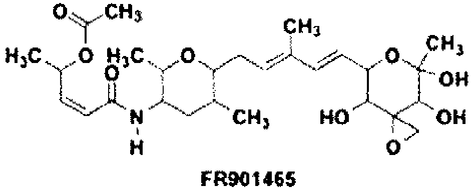
(ii) einer Verbindung mit einer Struktur 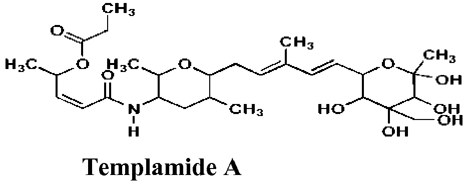
(iii) einer Verbindung mit einer Struktur 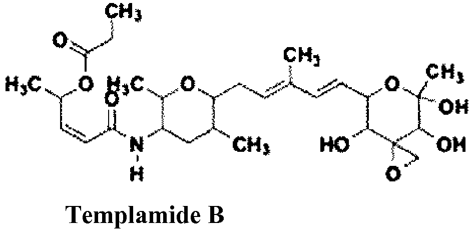
(iv) einer Verbindung mit einer Struktur 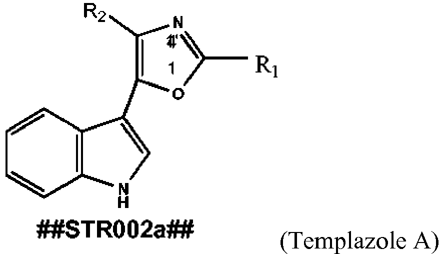 wobei R1 Isobutyl ist und R2 Carboxylsäuremethylester ist; und
(v) einer Verbindung mit einer Struktur 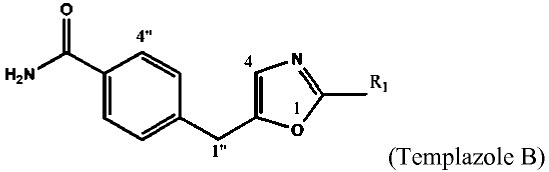
wobei R1 Isobutyl ist,
das wirksam ist zur Modulation des Schädlingsbefalls.

6. Verfahren nach Anspruch 5, wobei der Schädling ein Fungus ist.

7. Verfahren nach Anspruch 5, wobei der Schädling ein Insekt ist.

8. Verfahren nach Anspruch 5, wobei der Schädling ein monokotyles, Segge- oder dikotyles Unkraut ist.

9. Verfahren nach Anspruch 8, wobei das Verfahren das Auftreten und/oder Wachstum des monokotylen, Segge- oder dikotylen Unkrauts moduliert.

10. Verfahren nach einem der Ansprüche 5-9, wobei die Zusammensetzung umfassend den isolierten Stamm nach Anspruch 1 angewandt wird.

11. Verfahren nach einem der Ansprüche 5-9, wobei die Zusammensetzung umfassend die isolierte Verbindung mit der Struktur 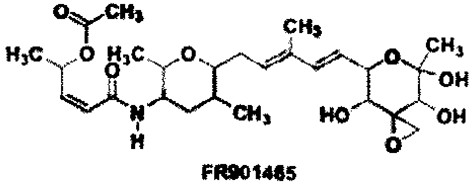 angewandt wird.

12. Verfahren nach einem der Ansprüche 5-9, wobei die Zusammensetzung umfassend die isolierte Verbindung mit der Struktur 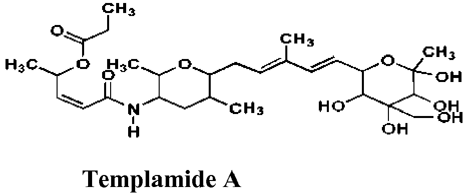 angewandt wird.

13. Verfahren nach einem der Ansprüche 5-9, wobei die Zusammensetzung umfassend die isolierte Verbindung mit der Struktur 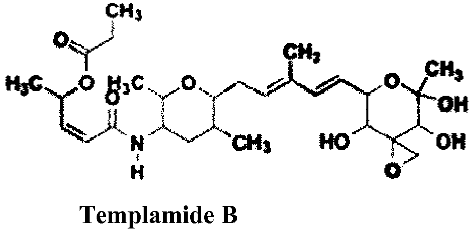 angewandt wird.

14. Verfahren nach einem der Ansprüche 5-9, wobei die Zusammensetzung umfassend die isolierte Verbindung mit der Struktur 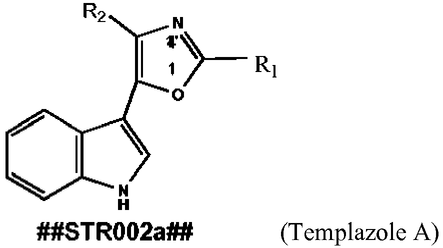 angewandt wird, wobei R1 Isobutyl ist und R2 Carboxylsäuremethylester ist.

15. Verfahren nach einem der Ansprüche 5-9, wobei die Zusammensetzung umfassend die isolierte Verbindung mit der Struktur 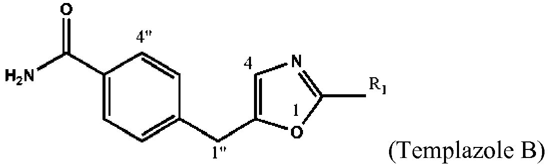 angewandt wird, wobei R1 Isobutyl ist.

16. Samen umfassend die Zusammensetzung umfassend den isolierten Stamm nach Anspruch 1 oder eine isolierte Verbindung mit pestizider Aktivität, ausgewählt aus
(i) einer Verbindung mit einer Struktur (FR901228) 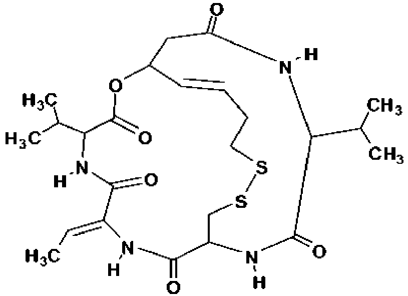
(ii) einer Verbindung mit einer Struktur 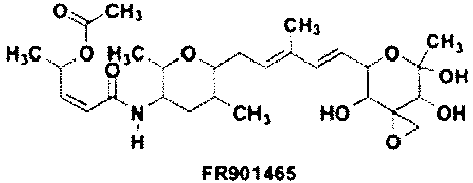
(iii) einer Verbindung mit einer Struktur 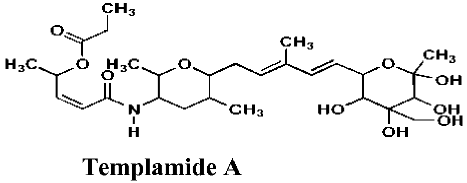
(iv) einer Verbindung mit einer Struktur 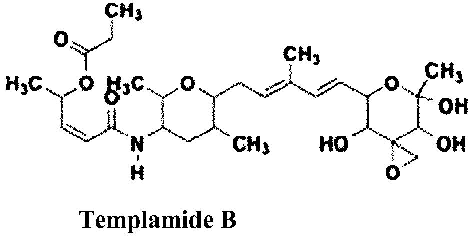
(v) einer Verbindung mit einer Struktur 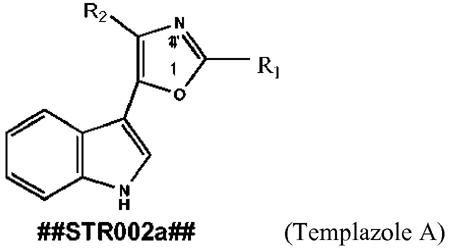 wobei R1 Isobutyl ist und R2 Carboxylsäuremethylester ist; und
(vi) einer Verbindung mit einer Struktur 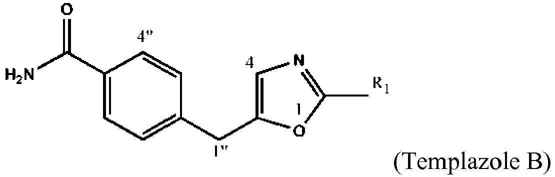 wobei R1 Isobutyl ist.

## Revendications

1. Souche isolée de *Burkholderia* A396 (No d'accès NRRL B-50319) qui possède les caractéristiques suivantes :
(A) une séquence de gêne d'ARN 16S comprenant les séquences sens ayant au moins 99% d'identité avec les séquences indiquées dans SEQ ID NO:8, 11, et 12 et les séquences antisens ayant au moins 99 % d'identité avec les séquences indiquées dans SEQ ID NO:9, 10, 13, 14 et 15 ;
(B) activité pesticide ;
(C) produit un composé pesticide choisi parmi
(i) un composé ayant une structure (FR901228) 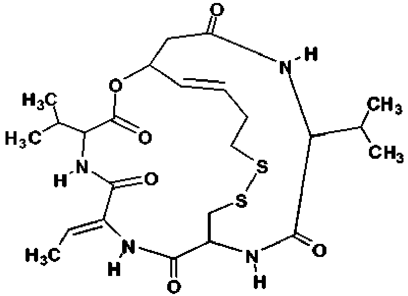
(ii) un composé ayant une structure 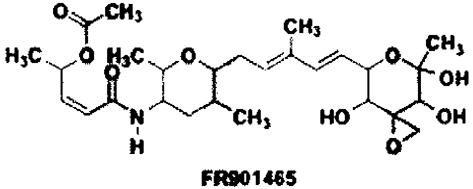
(iii) un composé ayant une structure 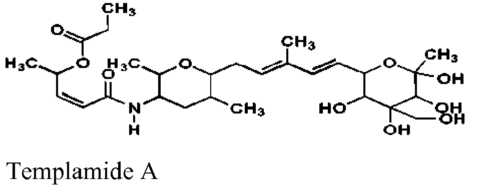
(iv) un composé ayant une structure 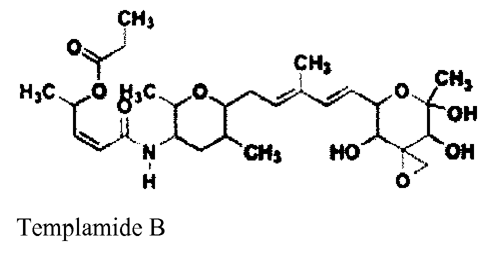
(v) un composé ayant une structure 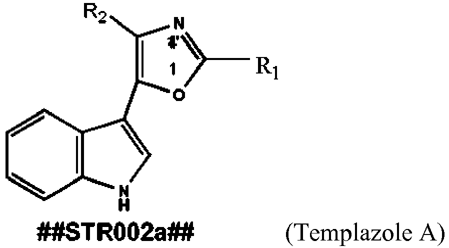 dans laquelle R1 est l'isobutyle et R2 est un ester méthylique d'acide carboxylique ; et
(vi) un composé ayant une structure 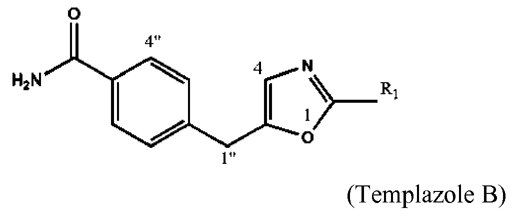 dans laquelle R1 est l'isobutyle ;
(D) est non pathogène pour les animaux vertébrés ; et
(E) est sensible à kanamycine, chloramphénicol, ciprofloxacine, pipéracilline, imipénem, et à une combinaison de sulphaméthoxazole et de triméthoprim.

2. Composé isolé ayant une activité pesticide pouvant être obtenu à partir d'une espèce *Burkholderia* choisie parmi
(i) un composé ayant une structure 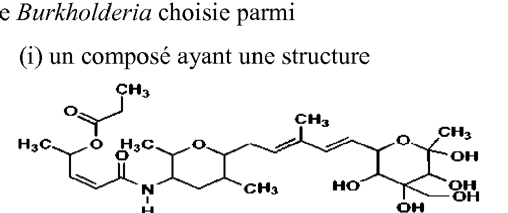
(ii) un composé ayant une structure 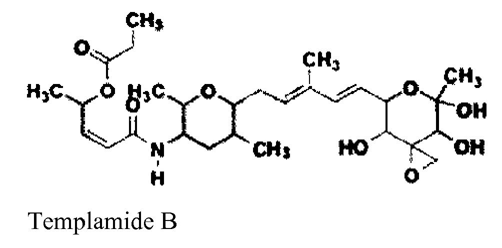
(iii) un composé ayant une structure 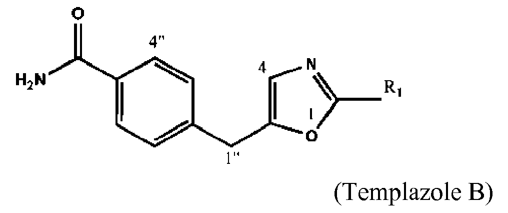 dans laquelle R1 est l'isobutyle.

3. Procédé de production d'un composé de la revendication 2, lequel procédé comprend la culture de la souche de la revendication 1 et la production dudit composé.

4. Composition comprenant la souche isolée de la revendication 1 ou un composé isolé de la revendication 2, la composition ayant une activité pesticide.

5. Procédé de modulation de l'infestation par des ravageurs dans un végétal comprenant l'application au végétal et/ou à ses graines et/ou au substrat utilisé pour cultiver ledit végétal, d'une quantité d'une composition comprenant la souche isolée de la revendication 1, ou d'une composition comprenant le composé isolé ayant l'activité pesticide choisi parmi
(i) un composé ayant une structure 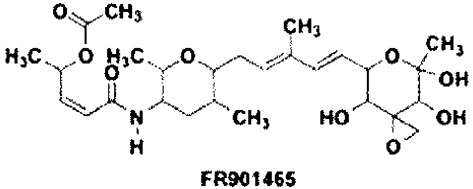
(ii)un composé ayant une structure 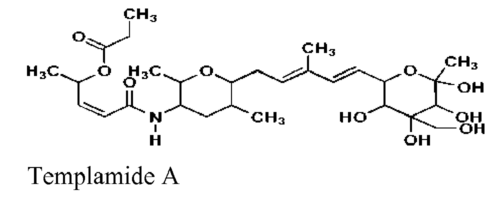
(iii) un composé ayant une structure 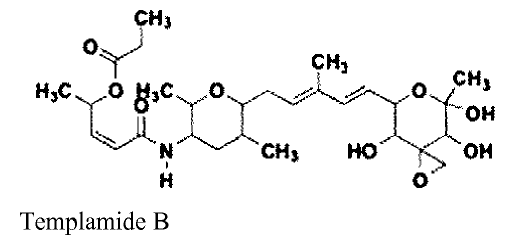
(iv) un composé ayant une structure 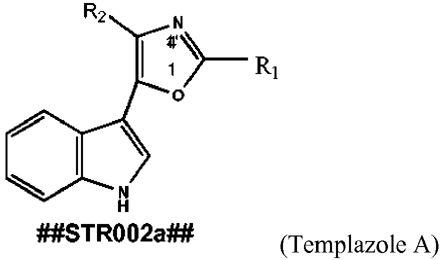 dans laquelle R1 est l'isobutyle et R2 est un ester méthylique d'acide carboxylique ; et
(v) un composé ayant une structure 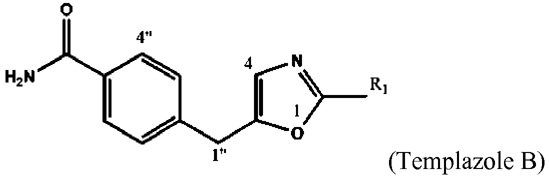
dans laquelle R1 est l'isobutyle,
efficace pour moduler ladite infestation par le ravageur.

6. Procédé selon la revendication 5, le ravageur étant un champignon.

7. Procédé selon la revendication 5, le ravageur étant un insecte.

8. Procédé selon la revendication 5, le ravageur étant une monocotylédone, un carex, ou une mauvaise herbe dicotylédone.

9. Procédé selon la revendication 8, le procédé modulant l'émergence et/ou la croissance des monocotylédones, carex ou mauvaises herbes dicotylédones.

10. Procédé selon l'une quelconque des revendications 5 à 9, la composition comprenant la souche isolée de la revendication 1 étant appliquée.

11. Procédé selon l'une quelconque des revendications 5 à 9, dans lequel la composition comprenant le composé isolé ayant la structure 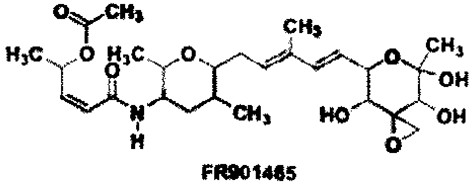 est appliquée.

12. Procédé selon l'une quelconque des revendications 5 à 9, dans lequel la composition comprenant le composé isolé ayant la structure 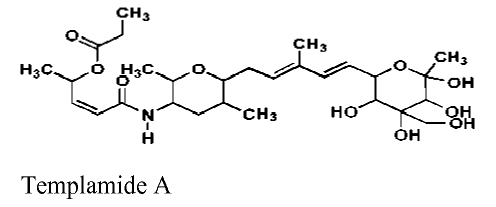 est appliquée.

13. Procédé selon l'une quelconque des revendications 5 à 9, dans lequel la composition comprenant le composé isolé ayant la structure 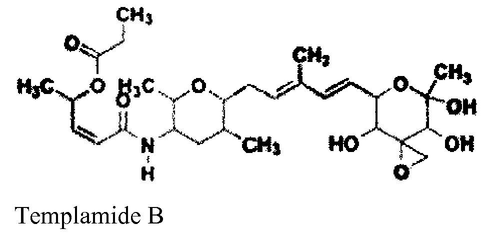 est appliquée.

14. Procédé selon l'une quelconque des revendications 5 à 9, dans lequel la composition comprenant le composé isolé ayant la structure 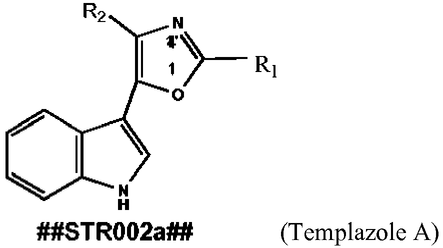 est appliquée, R1 étant l'isobutyle et R2 étant un ester méthylique d'acide carboxylique ; et

15. Procédé selon l'une quelconque des revendications 5 à 9, dans lequel la composition comprenant le composé isolé ayant la structure 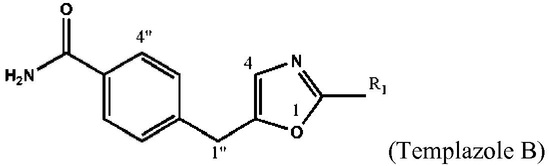 est appliquée, R1 étant l'isobutyle.

16. Semence comprenant la composition comprenant la souche isolée de la revendication 1 ou un composé isolé ayant une activité pesticide choisi parmi
(i) un composé ayant une structure (FR901228) 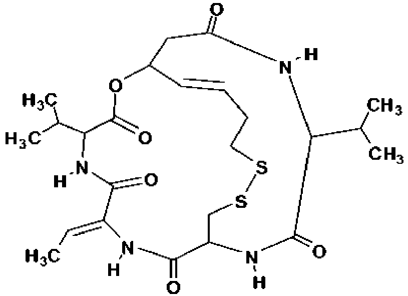
(ii) un composé ayant une structure 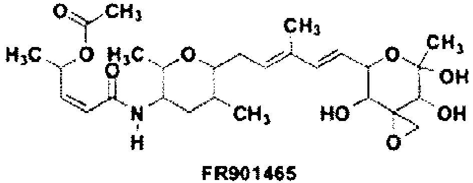
(iii) un composé ayant une structure 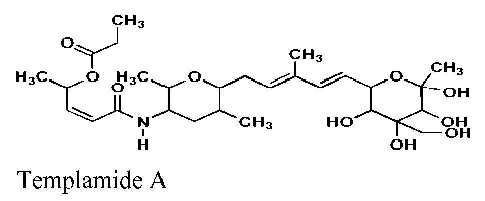
(iv) un composé ayant une structure 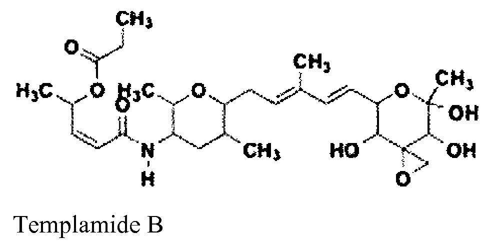
(v) un composé ayant une structure 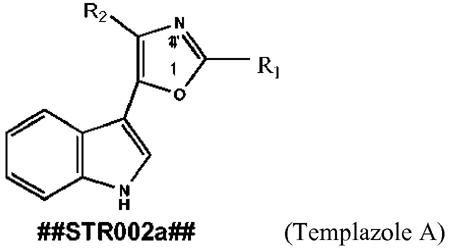 dans laquelle R1 est l'isobutyle et R2 est un ester méthylique d'acide carboxylique ; et
(vi) un composé ayant une structure 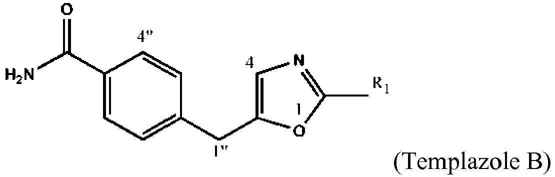 dans laquelle R1 est l'isobutyle.
